# EUROPEAN PATENT SPECIFICATION

(11) **EP 3 803 406 B1**
(45) Date of publication and mention of the grant of the patent: **01.07.2026**
(21) Application number: 19726029.2
(22) Date of filing: 28.05.2019
(51) Int. Cl.: G01N 33/68

(54) **HSP70 PROTEIN LEVELS IN PBMC SAMPLES AS BIOMARKER FOR DISEASE**
HSP70-PROTEIN-SPIEGEL IN PBMC-PROBEN ALS BIOMARKER FÜR KRANKHEITEN
TAUX DE PROTÉINE HSP70 DANS DES ÉCHANTILLONS DE PBMC EN TANT QUE BIOMARQUEUR D'UNE MALADIE

(30) Priority: 28.05.2018 EP 18174576
(43) Date of publication of application: 14.04.2021
(73) Proprietor: Zevra Denmark A/S, 2000 Frederiksberg (DK)
(72) Inventor: INGEMANN, Linda, 2870 Dyssegård (DK); KIRKEGAARD JENSEN, Thomas, 2610 Rødovre (DK)
(74) Representative: Høiberg P/S
(86) International application number: PCT/EP2019/063854
(87) International publication number: WO 2019/229078

(56) References cited:
- LOREDANA BASIRIC�2 ET AL: "Cellular thermotolerance is associated with heat shock protein 70.1 genetic polymorphisms in Holstein lactating cows", CELL STRESS AND CHAPERONES ; A COMPREHENSIVE JOURNAL OF STRESS BIOLOGY AND MEDICINE, SPRINGER NETHERLANDS, DORDRECHT, vol. 16, no. 4, 28 January 2011 (2011-01-28), pages 441 - 448, XP019918574, ISSN: 1466-1268, DOI: 10.1007/S12192-011-0257-7
- HEINE L ET AL: "Induction of the 68 kDa major heat-shock protein in different Theileria annulata- and virus-transformed bovine lymphoblastoid cell lines", VETERINARY IMMUNOLOGY AND IMMUNOPATHOLOGY, ELSEVIER, AMSTERDAM, NL, vol. 33, no. 3, 1 August 1992 (1992-08-01), pages 271 - 277, XP023888388, ISSN: 0165-2427, [retrieved on 19920801], DOI: 10.1016/0165-2427(92)90187-U
- DEB RAJIB ET AL: "Promoter variants at AP2 box region of Hsp70.1 affect thermal stress response and milk production traits in Frieswal cross bred cattle", GENE, ELSEVIER AMSTERDAM, NL, vol. 532, no. 2, 20 September 2013 (2013-09-20), pages 230 - 235, XP028760090, ISSN: 0378-1119, DOI: 10.1016/J.GENE.2013.09.037
- JEAN-CHRISTOPHE BAMBOU ET AL: "Effect of heat challenge on peripheral blood mononuclear cell viability: comparison of a tropical and temperate pig breed", TROPICAL ANIMAL HEALTH AND PRODUCTION, KLUWER ACADEMIC PUBLISHERS, DO, vol. 43, no. 8, 14 April 2011 (2011-04-14), pages 1535 - 1541, XP019967838, ISSN: 1573-7438, DOI: 10.1007/S11250-011-9838-9
- BOIOCCHI CHIARA ET AL: "Are Hsp70 protein expression and genetic polymorphism implicated in multiple sclerosis inflammation?", JOURNAL OF NEUROIMMUNOLOGY, ELSEVIER SCIENCE PUBLISHERS BV, NL, vol. 268, no. 1, 16 January 2014 (2014-01-16), pages 84 - 88, XP028662670, ISSN: 0165-5728, DOI: 10.1016/J.JNEUROIM.2014.01.004
- THOMAS KIRKEGAARD ET AL: "Heat shock protein-based therapy as a potential candidate for treating the sphingolipidoses", SCIENCE TRANSLATIONAL MEDICINE, vol. 8, no. 355, 7 September 2016 (2016-09-07), US, pages 355ra118 - 1, XP055390260, ISSN: 1946-6234, DOI: 10.1126/scitranslmed.aad9823
- SCHULTZ MARK L ET AL: "Lysosome and endoplasmic reticulum quality control pathways in Niemann-Pick type C disease", BRAIN RESEARCH, ELSEVIER, AMSTERDAM, NL, vol. 1649, 26 March 2016 (2016-03-26), pages 181 - 188, XP029761301, ISSN: 0006-8993, DOI: 10.1016/J.BRAINRES.2016.03.035
- HUNTER-LAVIN CLAIRE ET AL: "Hsp70 release from peripheral blood mononuclear cells", BIOCHEMICAL AND BIOPHYSICAL RESEARCH COMMUNICATIONS, vol. 324, no. 2, 2 October 2004 (2004-10-02), pages 511 - 517, XP028808429, ISSN: 0006-291X, DOI: 10.1016/J.BBRC.2004.09.075
- THOMAS KIRKEGAARD ET AL: "Heat shock protein-based therapy as a potential candidate for treating the sphingolipidoses", SCIENCE TRANSLATIONAL MEDICINE, vol. 8, no. 355, 7 September 2016 (2016-09-07), US, pages 355ra118 - 1, XP055390260, ISSN: 1946-6234, DOI: 10.1126/scitranslmed.aad9823
- SCHULTZ MARK L ET AL: "Lysosome and endoplasmic reticulum quality control pathways in Niemann-Pick type C disease", BRAIN RESEARCH, ELSEVIER, AMSTERDAM, NL, vol. 1649, 26 March 2016 (2016-03-26), pages 181 - 188, XP029761301, ISSN: 0006-8993, DOI: 10.1016/J.BRAINRES.2016.03.035
- HUNTER-LAVIN CLAIRE ET AL: "Hsp70 release from peripheral blood mononuclear cells", BIOCHEMICAL AND BIOPHYSICAL RESEARCH COMMUNICATIONS, vol. 324, no. 2, 2 October 2004 (2004-10-02), pages 511 - 517, XP028808429, ISSN: 0006-291X, DOI: 10.1016/J.BBRC.2004.09.075

## Description

### Technical field

The present invention relates to the finding that Heat Shock Protein 70 (Hsp70) is markedly reduced in peripheral blood mononuclear cell (PBMC) patient samples of the lysosomal storage disease NPC, and hence the previously observed reduced level of Hsp70 observed in pathologically afflicted disease tissue, including brain and liver tissue, directly translate into low levels of Hsp70 protein in PBMC samples, providing a simple detection means of Hsp70 levels.

### Background

Hsp70 proteins, one of the most extensively studied families of heat shock proteins (HSPs), are synthesized in all eukaryotic cells. Hsp70 proteins have a broad spectrum of chaperone functions and provide the normal course of many intracellular processes. In addition, they are involved in the cell resistance against stress; in particular, they prevent protein aggregation and facilitate the elimination of proteins damaged under stress conditions.

Hsp70 proteins are induced by stress and heat shock, and multiple reports have documented a correlation between Hsp70 expression and disease or other conditions of stress and exercise. For instance, elevated circulating levels of Hsp70 (in plasma or serum) are identified in cardiovascular disease (heart failure after acute myocardial infarction, peripheral artery disease), cancer patients (e.g. small cell lung cancer, cholangiocarcinoma, head and neck cancer, pancreatic cancer), preeclampsia and pathological pregnancies, gestational diabetes mellitus, polycystic ovary syndrome, inflammatory conditions, asthma and frailty in elderly patients. Such elevated circulating levels Hsp70 levels are found to predict disease progression and an unfavourable clinical outcome.

Under certain pathological conditions the protein quality control machinery is not sufficient to prevent the accumulation of misfolded proteins. Hsp70 functions as a chaperone and protects neurons from protein aggregation and toxicity. A common feature among various neurodegenerative diseases, including Alzheimer disease (AD), Parkinson disease (PD), amyotrophic lateral sclerosis (ALS), and the inheritable polyglutamine (PolyQ) diseases (e.g., Huntington disease (HD); spinocerebellar ataxia (SCA) type 1, 2, 3, 6, 7, and 17; spinobulbar muscular atrophy (SBMA); dentatorubral pallidoluysian atrophy (DRPLA)) is the accumulation and deposition of misfolded proteins in the brain (inside and outside neurons) and selective neuronal loss in the central nervous system (CNS). For all of these conformational/misfolding diseases, misfolded proteins are considered a common therapeutic target, and many studies have focused on the neuroprotective role of HSPs.

The worldwide incidence of neurodegenerative diseases is high. As neurodegenerative diseases disproportionately affect older individuals, disease-related morbidity has increased along with the general increase in longevity. An understanding of the underlying mechanisms that lead to neurodegeneration is key to identifying methods of prevention and treatment. Investigators have observed protective effects of HSPs induced by preconditioning, overexpression, or drugs in a variety of models of brain disease. Experimental data suggest that manipulation of the cellular stress response, including the provision of Hsp70, may offer strategies to protect the brain during progression of neurodegenerative disease.

The lysosomal storage diseases (LSD) are a rare group of diseases, characterized by the accumulation of substances in the lysosomal compartment and resulting destabilization hereof, with a resulting devastating effect for affected individuals. Substances accumulate in the lysosomal compartment due to deficiencies in the enzymes involved in their catabolism.

The majority of LSD patients are initially screened by an enzyme assay, if available, which is the most efficient method to arrive at a definitive diagnosis. In some families where the disease-causing mutation(s) is known and in certain genetic isolates, mutation analysis may be performed. As there may be numerous different mutations, sequencing of the gene encoding the particular affected protein/enzyme is sometimes necessary to confirm the diagnosis. Prenatal diagnosis may be useful when there is a known genetic risk factor.

LSDs include Niemann Pick disease, including types A, B and C. Historically, the diagnosis of Niemann Pick disease Type C (NPC) is made histopathologically, by both esterification studies and filipin staining of cultured skin fibroblasts, with most patients receiving a combination of different tests performed prior to this reliable, but costly and difficult, definitive investigation. These tests may have included: chitotriosidase measurements, white cell enzyme studies to exclude other lysosomal storage diseases, and fluorescent and electron microscopy of both bone marrow aspirate and liver biopsy specimens. Because of the difficulties with the filipin staining test, the most widely performed and accessible definitive diagnostic test is currently the sequencing of the NPC1 and NPC2 genes. Next-generation sequencers make this far easier to perform, especially if the genes concerned are included on a multi-gene panel appropriate for patients presenting with a certain disease phenotype - such as neonatal cholestatic jaundice, but this approach is not without limitations either. In 10% of patients only a single pathogenic mutation can be identified, and in some patients new mutations of uncertain clinical significance may be identified. Schultz *et al.* (Brain Research, Elsevier, Amsterdam, NL, vol. 1649 p. 181-188, 2016) has reviewed lysosome and endoplasmic reticulum pathways in NPC.

Hunter-Lavin et al. (Biochem. and Biophys. Res Comm, vol. 324 no. 2, p. 511-517, 2004) relates to Hsp70 in human serum and discloses Hsp70 release from PBMCs.

Basiricò et al. (Cell Stress and Chaperones 16:441-448, 2011) discloses the measurement of Hsp70.1 in PBMC of dairy cows by an ELISA assay. Heine et al. (Vet Immunol and Immunopat, 33, 271-277, 1992) analysed the expression of Hsp68 in PBMC from cattle by western blot. Deb et al. (Gene 532, p. 230-235, 2013) discloses detection of Hsp70.1 mRNA in PBMC of cattle. Bambou et al. (Trop Anim Health Prod 43:1535-1541, 2011) discloses measurement of Hsp70.2 mRNA expression in PBMC from pigs. Boiocchi et al. (J Neuroimmunol 268 p. 84-88, 2014) discloses an implication of HSP70-2 gene polymorphism in the development of multiple sclerosis (MS), and finds a lower Hsp70-2 protein content in PBMCs from patients with MS with the GG genotype.

It has been demonstrated that Hsp70 levels in the brain and liver of a Niemann Pick disease Type C (NPC) mouse model (*Npc1*^{*-*/*-*}) is lower than Hsp70 levels in the wild-type mouse organs (Kirkegaard, T. *et al.* 2016).

Thus, a number of diseases present with a reduced level of Hsp70 in affected tissues, such as the neurons of the CNS and PNS as well as the organs of afflicted individuals.

### Summary

The invention is defined by the claims. Any subject matter falling outside the scope of the claims is provided for information purposes only.

The references to methods of treatment in this description are to be interpreted as references to the compounds, pharmaceutical compositions and medicaments of the present invention for use in a method for the treatment of the human (or animal) body by therapy (or for diagnosis).

A number of diseases present with reduced level of Hsp70, and it would be highly useful to apply this finding of reduced Hsp70 levels as a general biomarker for aiding in identifying said diseases, or alternatively identifying the subset of patients with diseases co-presenting with a reduced level of Hsp70.

This finding may be applied in methods for diagnosing of patients with a disease presenting with a reduced level of Hsp70, regardless of the pathological consequences and symptoms of such reduced Hsp70, and alternatively identifying or diagnosing the subset of patients with diseases co-presenting with a reduced level of Hsp70. It may also find use in methods for monitoring of disease progression and efficacy of therapy in individuals with a disease presenting with a reduced level of Hsp70.

Thus, in addition to the measurement of Hsp70 levels, a further level of diagnosis is usually performed separately, simultaneously or subsequently in order to determine the specific disease presenting or co-presenting with a reduced level of Hsp70. Correct diagnosis is pivotal for enabling the correct treatment regimens, including those treatments which are approved and specific to the given pathology and opening for the possibility for testing combination treatment with therapies of Hsp70 supplementing and induction.

A reduced level of Hsp70 has been demonstrated in brain tissue and liver tissue samples from a Niemann Pick disease Type C mouse model. However, a biomarker found in tissues such as brain tissue and liver tissue samples is not considered applicable in terms of diagnosing of NPC, let alone any other disease presenting with a reduced level of Hsp70. Hence, other means for identifying diseases presenting with a reduced level of Hsp70 are warranted.

The present inventors have surprisingly found that specifically peripheral blood mononuclear cell (PBMC) samples from patients with Niemann Pick disease Type C contain substantially reduced or decreased levels of Heat Shock Protein 70 (Hsp70), as compared to healthy controls.

A PBMC sample can be easily obtained from an individual by simply drawing a blood sample, and performing a few simple steps of separation and isolation of PBMCs. Hence, the new observation that the low Hsp70 levels observed in pathologically afflicted disease tissue, including brain and liver tissue, directly translate into low levels of Hsp70 protein in PBMC samples, provides a simple, easy and facile detection means of Hsp70 levels.

It is an aspect of the present disclosure to provide
a method for diagnosing a disease selected from NPC and ALS in an individual, said method comprising the steps of:
a) providing a PBMC sample obtained from said individual,
b) detecting Hsp70 in said PBMC sample,
c) quantifying or determining the level of Hsp70 in said PBMC sample, and
d) optionally, classifying or determining whether or not the individual has, or is likely to have, a disease selected from NPC and ALS,
wherein said Hsp70 is HspA1A and HspA1B.

In a further aspect of the present disclosure there is provided a method for monitoring disease progression in an individual having a disease selected from NPC and ALS, said method comprising the steps of
i. providing one or more PBMC samples from said individual at two or more subsequent points in time,
ii. detecting Hsp70 in each of said PBMC samples, and
iii. quantifying or determining the level of Hsp70 in each of said PBMC samples,
wherein said Hsp70 is HspA1A and HspA1B.

In a further aspect of the present disclosure there is provided a method for monitoring efficacy of a therapy for treatment of a disease selected from NPC and ALS in an individual having a disease selected from NPC and ALS, said method comprising the steps of
a) providing one or more PBMC samples from said individual before, during and/or after said therapy has been applied, maintained, reduced or elevated,
b) detecting Hsp70 in each of said one or more PBMC samples, and
c) quantifying or determining the level of Hsp70 in each of said one or more PBMC samples,
wherein said Hsp70 is HspA1A and HspA1B.

### Description of Drawings

Figure 1. Quantification of the level of Hsp70 protein in PBMC samples isolated from individuals with Niemann Pick disease Type C (NPC) at a first and at a second clinical study visit, or from healthy individuals (controls). The level of Hsp70 was markedly reduced in PBMC samples obtained from NPC patients as compared with PBMC samples obtained from healthy controls, as determined by ELISA.
Figure 2. Comparison of the NPC-severity scale score (NPCCSS) with the Hsp70 level in PBMC samples obtained from individuals with Niemann Pick disease Type C. No correlation between Hsp70 level and NPCCSS was observed.
Figure 3. Comparison of the Hsp70 level in PBMC samples obtained from individuals with Niemann Pick disease Type C at a first and a second visit. The second clinical study visit was performed 6 to 14 months following the first clinical study visit. No change in Hsp70 level was observed over the time from clinical study visit 1 and 2.
Figure 4. HSP70 levels in NPC patients treated with arimoclomol incl. pretreatment.
Figure 5. HSP70 levels in NPC patients treated with arimoclomol.

### Detailed description

The present invention is based on the finding of decreased levels of Hsp70 in peripheral blood mononuclear cells (PBMC) samples from patients with Niemann Pick disease Type C, a lysosomal storage disease presenting with reduced levels of Hsp70 in pathologically afflicted disease tissue, including brain and liver tissue, as compared to a control.

Hsp70 proteins are involved in a wide range of cellular processes including protein folding and degradation of unstable cellular proteins as well as serving other cytoprotective roles. Thus, Hsp70 serves several important roles in cell homeostasis, and a number of diseases present with a reduced level of Hsp70, including lysosomal storage diseases, neurodegenerative diseases, and some neuromuscular and muscular diseases.

LSD are a group of rare inherited metabolic disorders that result from defects in lysosomal function as a consequence of deficiency of a single lysosomal protein or enzyme required for the metabolism or transport of lipids, glycolipids, glycoproteins or mucopolysaccharides. Although each disorder results from different gene mutations that translate into a deficiency in protein activity, they all share a common biochemical characteristic - all lysosomal disorders originate from an abnormal accumulation of substances inside the lysosome. Lysosomal storage diseases affect mostly children and they often die at a young and unpredictable age, many within a few months or years of birth. Many other children die of this disease following years of suffering from various symptoms of their particular disorder.

Niemann Pick disease Type C (NPC) is a devastating lysosomal storage disease of the sphingolipidosis-type caused by mutations in either the *NPC1* or *NPC2* gene, resulting in a dysfunctional lysosomal compartment and aberrant accumulation of cholesterol, sphingosine and glycosphingolipids in multiple tissues. Identification of biomarkers to follow disease progression in blood samples has mainly been focused on cholesterol and its derivatives.

NPC also present with reduced levels of Hsp70 in brain and liver tissue, and now the inventors have identified that this finding translates into reduced levels of Hsp70 in peripheral blood mononuclear cells (PBMC) samples from NPC patients.

Patients with diseases presenting with a reduced level of Hsp70 may benefit from increasing the level of Hsp70. Thus, the present disclosure in one embodiment provides means for identifying patients with a disease presenting with a reduced level of Hsp70 selected from NPC and ALS, which individual may benefit from increasing the level of Hsp70.

In one embodiment increasing the level of Hsp70 comprises increasing the intracellular concentration of Hsp70 by amplifying Hsp70 gene expression.

In one embodiment increasing the level of Hsp70 comprises increasing the intracellular concentration of Hsp70 by administering Hsp70, or a functional fragment or variant thereof.

### The Heat Shock Protein 70 Family

Hsp70 proteins are involved in a wide range of cellular processes including protein folding and degradation of unstable cellular proteins as well as serving other cytoprotective roles. The common function of Hsp70 in these processes appears to be the binding of short hydrophobic segments in partially folded polypeptides, thereby facilitating proper folding and preventing aggregation. In eukaryotes, Hsp70 chaperones interact *in vivo* with different classes of proteins that serve to regulate critical steps of their functional cycle; amongst these the J-domain family protein Hsp40. Furthermore, additional partner proteins have been identified, some of which are linking Hsp70 to other chaperone systems such as the Hsp90 system.

### Members of the Human Hsp70 Family

Some of the important functions attributed to the molecular chaperones include import of proteins into cellular compartments, folding of proteins in the cytosol, endoplasmic reticulum and mitochondria, prevention of protein aggregation and refolding of misfolded proteins. At present the human Hsp70 family includes 15 members encoded by different genes. The Hsp70 genes and proteins may be referred to herein by their locus name. Reference to Hsp70 usually refers to the two major inducible Hsp70 family members with loci names HSPA1A and HSPA1B, but may also refer to the whole Hsp70 family in general as evident from the consensus of the text.

### HspA1A and HspA1B

The genes transcribed from the loci HSPA1A and HSPA1B are the two heat/stress-inducible Hsp70-genes and the majority of the literature concerning human Hsp70 refers to the proteins encoded by these two genes. The genes give rise to proteins consisting of 641 amino acids, having 99% homology to each other and were the first human Hsp70 family members to be cloned and characterized. The genes are linked in the MHC-class III complex at 6p21.3, are intron-less and with promoter regions containing HSEs, enabling them to bind HSFs and induce transcription in response to a variety of cellular assaults.

### HspA1L and HspA2

Two Hsp70 family members have been termed "chauvinist genes" because male germ cells favor their expression with strong prejudice. The *hspA1L* gene is a constitutively expressed intron-less Hsp70 family member located 4kb telomeric to the HSPA1A locus in the same MHC-class III complex on chromosome 6. It is expressed in low amounts both before and after heat shock but with the expression pattern favoring the testes in mouse, rat and humans with the 641 amino acids (aa) protein being 90% homologous to HspA1A. The *hspA2* gene was first isolated from a mouse genomic library and has later been shown to be constitutively expressed albeit in low levels in various tissues in the human body including skeletal muscle, ovary, small intestine, colon, brain, placenta and the kidneys, but highly expressed in testis. Its expression, or rather lack thereof, has been connected with abnormal human spermatogenesis and male *hspA2*^{*(-*/*-)*} mice are sterile. The gene is located on chromosome 14, giving rise to a 639 aa protein with 84% homology to HspA1A, although the exact location is subject to discussion as two papers have presented different loci positions - 14q24.1 vs. 14q22.

### HspA6 and HspA7

The *hspA6* and *hspA7* genes are heat inducible members of the Hsp70 family with no apparent counterparts in mice. They contain HSEs in their promoter-sites and the genes are intron-less. They are co-localized on chromosome 1 and are 94% homologous to each other in the nucleotide sequence. However, only HspA6 is functional as the *hspA7* gene harbors a single nucleotide insertion generating a premature stop codon at +1324. The HspA6 protein is 643 aa long and shows 77% homology to HspA1A and HspA1B.

### HspA5 and HspA9

The *hspA5* and *hspA9* genes are the two compartment-specific members of the Hsp70 family. The 655 aa HspA5 protein is located in the endoplasmic reticulum (ER) and facilitates folding and transport of newly synthesized proteins in this compartment. The protein is 64% homologous to HspA1A, the gene being located at 9q34. The 679 aa HspA9 protein is located in the mitochondria where it assists in folding of proteins after their transport across the mitochondrial membrane. HspA9 is located at 5q31.1, the protein being 52% homologous to HspA1A.

### HspA8

The cognate Hsp70 member known as Hsc70 is encoded by a gene named *hspA8* at 11q24, giving rise to a 646 aa protein with 86% homology to HspA1A, and is constitutively expressed in all tissues and cell lines. The protein is analogous to Hsp70 in its cellular functions, providing the required chaperoning under normal circumstances, but has also been ascribed a role in the un-coating of clathrin-coated vesicles as well as in chaperone-mediated autophagy. HspA3 and HspA4, HspA4L, HspA12A and HspA14 will not be further discussed herein.

**Table 1: List of the Human Hsp70 Gene Family. The genes are listed according to locus name, names used herein, chromosomal location (position), amino acid homology to HspA1A as well as alternative names often seen in the literature.**

| **Locus** | **Name Used herein, Gene/Protein** | **Position** | **% aa Homology to HSPA1A** | **Alternative Names** |
|---|---|---|---|---|
| HSPA1A | *hspA1A*/HspA1A (Hsp70) | 6p23.1 | 100 | Hsp70; Hsp72; Hsp70-1 |
| HSPA1B | *hspA1B*/HspA1B (Hsp70) | 6p23.1 | 99 | Hsp70; Hsp72; Hsp70-2 |
| HSPA1L | *hspa1L*/HspA1L | 6p23.1 | 90 | Hsp70-Hom; Hsp70t |
| HSPA2 | *hspA2*/HspA2 | 14q24.1 | 84 | Hsp70-3 |
| HSPA4 | *hspA4*/HspA4 | 5q31.1 | 31 | Hsp70RY; APG-2 |
| HSPA4L | *hspA4L*/*HspA4L* | | | APG1; OSP94 |
| HSPA5 | *hspA5*/HspA5 | 9q34 | 64 | BiP; GRP78 |
| HSPA6 | *hspA6*/HspA6 | 1q | 84 | Hsp70-6; Hsp70B' |
| HSPA7 | *hspA7*/HspA7 | 1q | - | Hsp70-7; Hsp70B |
| HSPA8 | *hspA8*/HspA8 (Hsc70) | 11q24 | 86 | Hsc70; Hsp73 |
| HSPA9 | *hspA9*/HspA9 | 5q31.1 | 52 | GRP75; PBP74; mtHsp75; mortalin; mot-2 |
| HSPA12A | *hspA12A*/*HspA12A* | | | KIAA0417 |
| HSPA14 | *hspA14*/*HspA14* | | | Hsp60; Hsp70L1 |

### Methods involving Hsp70 biomarker

The inventors have found that Hsp70 levels (HspA1A and HspA1B), are significantly lower in specifically PBMC samples obtained from NPC patients, a disease known to present with a reduced level of Hsp70 in pathologically afflicted disease tissue, including brain and liver tissue, as compared to healthy controls. Diseases presenting with a reduced level of Hsp70 selected from NPC and ALS may be alleviated by Hsp70 therapies and the present disclosure thus provides means for diagnosing patients having a disease selected from NPC and ALS which may benefit from treatment with Hsp70 therapies.

'Reduced' levels may be used interchangeably with decreased or lower levels herein. The Hsp70 levels may be reduced or lower as compared to a control, such as a healthy individual, or as compared to an different time point (e.g. first visit/sample compared to a later visit/sample).

The present methods allow for easy and facile detection of said Hsp70 levels in PBMC samples and enable the use of Hsp70 in PBMCs as a biomarker to provide a reliable and easy tool to identify diseases presenting with a reduced level of Hsp70, wherein said disease is selected from NPC and ALS.

The present methods in some embodiments comprise the detection and quantification of Hsp70 in PBMC sample obtained from an individual, wherein said Hsp70 is HspA1A and HspA1B.

Detection and quantification of Hsp70 in PBMC samples can thus be used in methods of diagnosing or identifying a patient with a disease presenting with a reduced level of Hsp70 selected from NPC and ALS.

Accordingly, a reduced amount of Hsp70 in a PBMC sample from an individual as compared to the amount detected in healthy controls is indicative of a disease presenting with a reduced level of Hsp70 selected from NPC and ALS; or indicative of the patient being eligible for Hsp70 therapies.

The present methods can also be used in methods of monitoring disease progression in an individual or patient with a disease presenting with a reduced level of Hsp70 selected from NPC and ALS.

The present methods can also be used in methods of monitoring efficacy of a treatment in an individual or patient with a disease presenting with a reduced level of Hsp70 selected from NPC and ALS.

### Methods of detecting Hsp70

In one embodiment Hsp70 is detected in a PBMC sample, comprising the steps of
a) providing a PBMC sample,
b) detecting Hsp70 in said PBMC sample, and
c) optionally quantifying or determining the level of Hsp70 in said PBMC sample,
wherein said Hsp70 is HspA1A and HspA1B.

In one embodiment, detecting and/or quantifying Hsp70 refer to the detection and quantification of Hsp70 protein, wherein said Hsp70 is HspA1A and HspA1B.

In one embodiment, detecting and/or quantifying Hsp70 refer to the detection and quantification of HspA1A and HspA1B, with no or little detection of HspA5 and/or HspA8.

In one embodiment, detecting and/or quantifying Hsp70 includes the detection of naturally occurring HspA1A and HspA1B and naturally occurring HspA1A and HspA1B variants. Naturally occurring HspA1A and HspA1B variants are known to the skilled person.

In one embodiment said Hsp70 is HspA1A (SEQ ID NOs: 1 and 2) and HspA1B (SEQ ID NOs: 4 and 5), or a functional fragment or variant thereof. In SEQ ID NO: 2 the initiator methionine (M at position 1) of SEQ ID NO: 1 is removed. In SEQ ID NO: 5 the initiator methionine (M at position 1) of SEQ ID NO: 4 is removed. *In vivo* this occurs by post-translational processing.

In one embodiment said step of detecting Hsp70 in a PBMC sample is performed *in vitro.*

Quantifying, in one embodiment, means determining the level of said Hsp70 protein present in the PBMC sample.

In one embodiment the level of Hsp70 is detected and/or quantified in a PBMC sample. In one embodiment said PBMC sample is obtained from or obtainable from an individual. In one embodiment said individual has, is suspected of having, is at risk of having or is likely to have, a disease selected from NPC and ALS. In one embodiment said individual has, is suspected of having, is at risk of having or is likely to have, the lysosomal storage disease Niemann Pick disease, type C (NPC). In one embodiment said individual has, is suspected of having, is at risk of having or is likely to have, the neurodegenerative disease ALS. The PBMC samples are disclosed in further detail herein elsewhere.

In one embodiment Hsp70 is detected in a PBMC sample comprising the steps of
a) providing a PBMC sample,
b) detecting Hsp70 in said PBMC sample, and
c) quantifying or determining the level of Hsp70 in said PBMC sample.
wherein said Hsp70 is HspA1A and HspA1B.

The present methods of detecting and optionally quantifying HspA1A and HspA1B in one embodiment do not exclude the detection of other Hsp70 proteins.

In one embodiment Hsp70 is detected in a PBMC sample, comprising the steps of
a) providing a PBMC sample,
b) detecting Hsp70 in said PBMC sample, and
c) quantifying or determining the level of Hsp70 in said PBMC sample,
wherein said Hsp70 is HspA1A and HspA1B,
wherein said PBMC sample is obtained from an individual having, suspected of having, at risk of having or likely to have a disease selected from NPC and ALS.

Also disclosed herein is detecting and optionally quantifying Hsp70 in a PBMC sample from an individual or patient with a disease selected from NPC and ALS before, during and/or after a therapy has been applied, maintained, reduced or elevated.

In one embodiment, said therapy comprises a therapy for inducing Hsp70 level and/or activity, such as or a bioactive agent capable of inducing the expression of Hsp70 and/or activity of Hsp70. In one embodiment, said therapy comprises a therapy for treatment of a disease selected from NPC and ALS, such as a bioactive agent effective in the treatment of a disease selected from NPC and ALS. Therapies and bioactive agents are disclosed herein elsewhere.

In one embodiment said PBMC sample from an individual comprises
i) one or more PBMC samples obtained from an individual having a disease selected from NPC and ALS before a therapy has been applied, maintained, reduced or elevated;
ii) one or more PBMC samples obtained from an individual having a disease selected from NPC and ALS during a therapy; and/or
iii) one or more PBMC samples obtained from an individual having a disease selected from NPC and ALS after a therapy has been applied, maintained, reduced or elevated; and/or;
iv) one or more PBMC samples obtained from an individual having a disease selected from NPC and ALS before and after a therapy has been applied, maintained, reduced or elevated.

Also disclosed herein is detecting and optionally quantifying Hsp70 in a PBMC sample from an individual having a disease selected from NPC and ALS at subsequent points in time to monitor disease progression, wherein said Hsp70 is HspA1A and HspA1B.

Also disclosed herein is detecting and optionally quantifying Hsp70 in a PBMC sample from an individual suspected of having a disease selected from NPC and ALS, to diagnose said disorder, wherein said Hsp70 is HspA1A and HspA1B.

Means for providing a PBMC sample, the nature of the PBMC sample and means for detection of Hsp70 and for quantifying Hsp70, wherein said Hsp70 is HspA1A and HspA1B, are disclosed herein elsewhere.

### Detection and quantification of Hsp70

The methods disclosed herein comprise one or more steps of detecting Hsp70 in a PBMC sample, including the steps of
a) providing one or more PBMC samples obtained from an individual,
b) detecting Hsp70 in said PBMC sample, and
c) optionally quantifying or determining the level of Hsp70 in said PBMC sample.
wherein said Hsp70 is HspA1A and HspA1B.

Hsp70 in PBMC according to the present disclosure may be detected at the DNA level, the RNA/mRNA level and/or the protein level. In a preferred embodiment, Hsp70 is detected at the protein level.

Detection and quantification of Hsp70, wherein said Hsp70 is HspA1A and HspA1B,can be performed by several methods known in the art, and may include one or multiple steps. Detection and quantification of Hsp70, wherein said Hsp70 is HspA1A and HspA1B, may be performed by any method known to the skilled person.

In one embodiment the steps of b) detecting Hsp70, wherein said Hsp70 is HspA1A and HspA1B, in said PBMC sample is performed by subjecting the PBMC sample to one or more steps of detection and/or quantification.

In one embodiment, detection and quantification of Hsp70 protein, wherein said Hsp70 is HspA1A and HspA1B, is performed by a Spectrometry methods or an Antibody dependent method.

In one embodiment, detection and quantification of Hsp70 protein, wherein said Hsp70 is HspA1A and HspA1B, is performed by a method selected from the group consisting of enzyme-linked immunosorbent assay (ELISA), western blotting, Protein immunoprecipitation, Immunoelectrophoresis, Protein immunostaining, High-performance liquid chromatography (HPLC) and Liquid chromatography-mass spectrometry (LC/MS).

In one embodiment, detection and quantification of Hsp70 protein, wherein said Hsp70 is HspA1A and HspA1B, is performed using mass spectrometry. In one embodiment, detection and quantification of Hsp70 protein in a dried blood spot (DBS) sample is performed using mass spectrometry.

Dried blood spot testing (DBS) is a form of biosampling, well-known to a person of skill in the art, where blood samples, such as 70µL of whole blood per spot, are blotted and dried on filter paper. The dried samples can easily be shipped to an analytical laboratory and analyzed using various methods, such as the methods mentioned herein, for example mass spectrometry. In one embodiment, the total protein is extracted from the blood spots soaked in phosphate buffered saline (PBS). In one embodiment, the protein is further subjected to trypsin digest, purified and analyzed by mass spectrometry.

In one embodiment, detection and quantification of Hsp70 protein, wherein said Hsp70 is HspA1A and HspA1B, is performed by means of enzyme-linked immunosorbent assay (ELISA).

In one embodiment the step of b) detecting Hsp70 in said PBMC sample and/or the step c) optionally quantifying or determining the level of Hsp70 in said PBMC sample, comprises one or more steps of
i) lysis of the PBMC sample, and/or
ii) immobilization of Hsp70 protein present in the lysed PBMC sample, and/or
iii) binding of primary antibodies to said immobilized Hsp70 protein, and/or
iv) binding of secondary antibodies to the primary antibody of iii), and/or
v) binding of streptavidin conjugates to the secondary antibody, to provide a streptavidin conjugate, and/or
vi) visualization of the primary antibody bound to Hsp70 protein, and/or
vii) visualization of the secondary antibody,
wherein said Hsp70 is HspA1A and HspA1B.

In one embodiment, the primary antibody of iii) is covalently associated with a biotin molecule to allow binding of the streptavidin conjugate of said secondary antibody.

In one embodiment, said primary antibody, said secondary antibody or said streptavidin conjugate of v) is covalently associated with an enzyme or a fluorescent probe.

Step vi) and vii) visualization of the primary antibody bound to Hsp70 or the secondary antibody bound to the primary antibody may be performed by colorimetric detection, chemiluminescent detection, radioactive detection, electrochemical detection or fluorescent detection. In one embodiment the visualization comprises conversion of one or more substrates by an enzyme covalently associated with said primary antibody bound to Hsp70, said secondary antibody bound to the primary antibody or said streptavidin conjugate bound to the primary antibody.

In one embodiment, the enzyme covalently associated with said primary antibody bound to Hsp70, said secondary antibody bound to the primary antibody or said streptavidin conjugate is horse radish peroxidase (HRP). In one embodiment, said substrate is selected from the group consisting of hydrogen peroxide, luminol, tetramethylbenzidine.

In one embodiment, the step of b) detecting Hsp70 in said PBMC sample and/or the step c) optionally quantifying or determining the level of Hsp70 in said PBMC sample further comprises washing steps in between the steps of lysis, immobilization, binding, and visualization.

ELISA typically is suitable to measure presence and/or amount of a given protein in a sample. Hence ELISA is suitable for measuring the presence and/or amount of Hsp70 in a PBMC sample.

In one embodiment the step of b) detecting Hsp70 in said PBMC sample and/or the step c) optionally quantifying or determining the level of Hsp70 in said PBMC sample, comprises one or more steps of detecting Hsp70 directly (e.g. by ELISA).

In one embodiment, a calibrated standard is used for quantification of Hsp70 present in the PBMC sample.

In one embodiment the calibrated standard for quantification is used at different concentrations.

In one embodiment, detection and quantification of Hsp70 mRNA is performed by a method selected from the group consisting of Northern blot, ribonuclease protection assay (RPA), and real-time polymerase chain reaction (RT-PCR).

### Methods of diagnosing

It is an aspect of the present disclosure to provide a method for diagnosing a disease selected from NPC and ALS in an individual, said method comprising the step of detecting Hsp70 in a PBMC sample obtained from or obtainable from an individual, wherein said Hsp70 is HspA1A and HspA1B.

In one embodiment said method for diagnosing a disease selected from NPC and ALS further comprises the step of quantifying or determining the level of Hsp70 present in the PBMC sample.

In one embodiment the level of the Hsp70 present in the PBMC sample is indicative of whether or not the individual has (or is suffering from) a disease selected from NPC and ALS, or whether or not the individual is likely to have or at risk of having (or suffering from) a disease selected from NPC and ALS.

The methods may be used to confirm a suspected diagnosis, for example if there is a suspicion that the individual has or suffers from a disease selected from NPC and ALS, based e.g. on family history, or on the presence of symptoms indicative of an LSD. The methods may be used in addition to known methods of diagnosing a disease selected from NPC and ALS.

It is thus an aspect of the present disclosure to provide a method for diagnosing a disease selected from NPC and ALS in an individual, said method comprising the steps of
a) providing a PBMC sample obtained from said individual,
b) detecting Hsp70 in said PBMC sample, and
c) quantifying or determining the level of Hsp70 in said PBMC sample,
wherein said Hsp70 is HspA1A and HspA1B.

In one embodiment the level of the Hsp70 present in the PBMC sample is indicative of whether or not the individual has (or is suffering from) a disease selected from NPC and ALS, or whether or not the individual is likely to have or at risk of having (or suffering from) a disease selected from NPC and ALS.

Also disclosed is a method for diagnosing a disease selected from NPC and ALS in an individual, said method comprising the steps of
a) providing a PBMC sample obtained from said individual,
b) detecting Hsp70 in said PBMC sample,
c) quantifying or determining the level of Hsp70 in said PBMC sample, and
d) classifying or determining whether or not the individual has, or is likely to have, a disease selected from NPC and ALS,
wherein said Hsp70 is HspA1A and HspA1B.

In one embodiment said PBMC sample is obtained from or obtainable from an individual.

Said method for diagnosing a disease selected from NPC and ALS comprise detecting and quantifying or determining the level of
i) HspA1A and HspA1B.

In one embodiment, step d) classifying or determining whether or not the individual has, or is likely to have, a disease selected from NPC and ALS, is a step of classifying or determining the individual as having, or likely to have a disease selected from NPC and ALS.

Reference to 'the sample' or 'a sample' herein will refer to a PBMC sample from the individual having or suspected of having a disease selected from NPC and ALS, unless otherwise specified. In contrast, a sample from a healthy control will be referred to as a PBMC sample from a healthy control or control sample.

In one embodiment said step d) of classifying or determining the individual as having, or likely to have, a disease selected from NPC and ALS, comprises determining the level of said Hsp70 in the PBMC sample as compared to the levels in a PBMC sample obtained or obtainable from a healthy control. A healthy control in the present context is an individual who does not have, or is not suspected of having, a disease selected from NPC and ALS. Preferably the healthy control also does not present with any other apparent disease. In one embodiment, the healthy control can be of any age. In one embodiment, the healthy control is an age-matched control. In one embodiment, the healthy control is below the age of 30, such as below 29, such as below 28, such as below 27, such as below 26, such as below 25, such as below 24, such as below 23, such as below 22, such as below 21, such as below 20, such as below 19, such as below the age of 18. In one embodiment, the healthy control is below the age of 20. In one embodiment, the healthy control is of age from about 4 to about 18. In one embodiment, the healthy control is of age from about 2 to about 4.

In one embodiment a decreased level of Hsp70 in the PBMC sample as compared to levels in a healthy control is indicative of the individual having, likely to have or at risk of having, a disease selected from NPC and ALS.

In one embodiment a level of Hsp70 in the PBMC sample which is comparable to or equal to the level in a healthy control is indicative of the individual not having a disease selected from NPC and ALS.

In one embodiment a level of Hsp70 in the PBMC sample which is higher than the level in a healthy control is indicative of the individual not having a disease selected from NPC and ALS.

In one embodiment the individual is likely to have a disease selected from NPC and ALS if
i) the level of HspA1A and HspA1B in the PBMC sample is lower than the level found in healthy controls, or undetectable.

In one embodiment the individual is likely to have a disease selected from NPC and ALS if the level of Hsp70, wherein said Hsp70 is HspA1A and HspA1B, in the PBMC sample is 1 to 1000 times lower than the level found in healthy controls, such as 1 to 2 times, 2 to 3 times, 3 to 4 times, 4 to 5 times, 5 to 6 times, 6 to 7 times, 7 to 8 times, 8 to 9 times, 9 to 10 times, 10 to 11 times, 11 to 12 times, 12 to 13 times, 13 to 14 times, 14 to 15 times, 15 to 16 times, 16 to 17 times, 17 to 18 times, 18 to 19 times, 19 to 20 times, 20 to 25 times, 25 to 30 times, 30 to 35 times, 35 to 40 times, 40 to 45 times, 45 to 50 times, 50 to 75 times, 75 to 100 times, 100 to 150 times, 150 to 200 times, 200 to 250 times, 250 to 300 times, 300 to 400 times, 400 to 500 times, 500 to 750 times, 750 to 1000 times lower than the level found in a healthy control, or undetectable.

In one embodiment the individual is likely to have a disease selected from NPC and ALS if the level of HspA1A and HspA1B in the PBMC sample is 1 to 1000 times lower than the level found in healthy controls, such as 1 to 2 times, 2 to 3 times, 3 to 4 times, 4 to 5 times, 5 to 6 times, 6 to 7 times, 7 to 8 times, 8 to 9 times, 9 to 10 times, 10 to 11 times, 11 to 12 times, 12 to 13 times, 13 to 14 times, 14 to 15 times, 15 to 16 times, 16 to 17 times, 17 to 18 times, 18 to 19 times, 19 to 20 times, 20 to 25 times, 25 to 30 times, 30 to 35 times, 35 to 40 times, 40 to 45 times, 45 to 50 times, 50 to 75 times, 75 to 100 times, 100 to 150 times, 150 to 200 times, 200 to 250 times, 250 to 300 times, 300 to 400 times, 400 to 500 times, 500 to 750 times, 750 to 1000 times lower than the level found in a healthy control, or undetectable.

In one embodiment 'undetectable' means that no signal is observed, such as no signal above the baseline noise. In one embodiment 'undetectable' means that the level of Hsp70 is undetectable in the PBMC sample.

In one embodiment said step d) of classifying or determining the individual as having, or likely to have, a disease selected from NPC and ALS, comprises determining if the amount of Hsp70 in said PBMC sample is below a predefined cut-off value, or undetectable.

If the amount of Hsp70 is below said cut-off value, the individual has or is likely to have a disease selected from NPC and ALS. If the amount of Hsp70 is equal to or above said cut-off value, the individual does not have or is not likely to have a disease selected from NPC and ALS.

In one embodiment said step d) of classifying or determining the individual as *not* having, or not likely to have, a disease selected from NPC and ALS, comprises determining if the amount of Hsp70 in said PBMC sample is equal to or above a predefined cut-off value.

Said cut-off values are determined based on the value in healthy controls, and compared to the value in individuals with a disease selected from NPC and ALS.

In one embodiment said cut-off values are determined based on the value in healthy controls compared to the value in patients with Niemann Pick disease Type C.

A cut-off value for a Hsp70, wherein said Hsp70 is HspA1A and HspA1B, of 5000 pg/mL PBMC means that a value of 5000 pg/mL Hsp70 or less is indicative of the individual having, or likely to have, a disease selected from NPC and ALS.

In one embodiment the individual has or is likely to have a disease selected from NPC and ALS if the amount of Hsp70, wherein said Hsp70 is HspA1A and HspA1B, in said PBMC sample is 7500 pg/mL or less, such as 7000 pg/mL or less, such as 6500 pg/mL or less, such as 6000 pg/mL or less, such as 5500 pg/mL or less, such as 5000 pg/mL or less, such as 4500 pg/mL or less, such as 4000 pg/mL or less, such as 3500 pg/mL or less, such as 3000 pg/mL or less, such as 2500 pg/mL or less, such as 2000 pg/mL or less, such as 1500 pg/mL or less, such as 1000 pg/mL PBMC or less.

In one embodiment the individual has or is likely to have a disease selected from NPC and ALS if the amount of HspA1A and HspA1B in said PBMC sample is 7500 pg/mL or less, such as 7000 pg/mL or less, such as 6500 pg/mL or less, such as 6000 pg/mL or less, such as 5500 pg/mL or less, such as 5000 pg/mL or less, such as 4500 pg/mL or less, such as 4000 pg/mL or less, such as 3500 pg/mL or less, such as 3000 pg/mL or less, such as 2500 pg/mL or less, such as 2000 pg/mL or less, such as 1500 pg/mL or less, such as 1000 pg/mL PBMC or less.

Conversely, an individual is classified as not having or not likely to have a disease selected from NPC and ALS if the amount of Hsp70, wherein said Hsp70 is HspA1A and HspA1B, in said PBMC sample is above 5000 pg/mL, such as above 5500 pg/mL, such as above 6000 pg/mL, such as above 6500 pg/mL, such as above 7000 pg/mL, such as above 7500 pg/mL, such as above 8000 pg/mL, such as above 8500 pg/mL, such as above 9000 pg/mL, such as above 9500 pg/mL, such as above 10000 pg/mL, such as above 10500 pg/mL, such as above 11000 pg/mL, such as above 11500 pg/mL, such as above 12000 pg/mL, such as above 12500 pg/mL PBMC.

In one embodiment an individual is classified as not having or not likely to have a disease selected from NPC and ALS if the amount of HspA1A and HspA1B in said PBMC sample is above 5000 pg/mL, such as above 5500 pg/mL, such as above 6000 pg/mL, such as above 6500 pg/mL, such as above 7000 pg/mL, such as above 7500 pg/mL, such as above 8000 pg/mL, such as above 8500 pg/mL, such as above 9000 pg/mL, such as above 9500 pg/mL, such as above 10000 pg/mL, such as above 10500 pg/mL, such as above 11000 pg/mL, such as above 11500 pg/mL, such as above 12000 pg/mL, such as above 12500 pg/mL PBMC.

Furthermore, an individual is classified as having or likely to have a disease selected from NPC and ALS if the amount of said Hsp70 in said PBMC sample is undetectable.

The diagnosis may be confirmed or infirmed using methods otherwise known in the art, or by repeating the diagnosis methods disclosed herein.

In one embodiment the present methods further comprises applying, maintaining, reducing, elevating or not applying a therapy based on whether or not the subject has, or is at risk of having a disease selected from NPC and ALS.

In one embodiment the method for diagnosing a disease selected from NPC and ALS in an individual comprises the step of e) administering a therapy for treatment of a disease selected from NPC and ALS to the patient diagnosed with said disease selected from NPC and ALS.

### Methods of diagnosing and treating

It is also an aspect of the present disclosure to provide a method for diagnosing and treating a disease selected from NPC and ALS in an individual, said method comprising the steps of
a) providing a PBMC sample obtained from said individual,
b) detecting Hsp70 in said PBMC sample,
c) quantifying or determining the level of Hsp70 in said PBMC sample,
d) classifying or determining whether or not the individual has, or is likely to have disease selected from NPC and ALS, and
e) administering a therapy for treatment of said disease selected from NPC and ALS to the individual,
wherein said Hsp70 is HspA1A and HspA1B.

In one embodiment step e) of administering a therapy for treatment of a disease selected from NPC and ALS to the individual comprises administering an effective amount of a bioactive agent to said individual, wherein said bioactive agent is effective for said disease selected from NPC and ALS.

It is also an aspect of the present disclosure to provide a method of treating an individual with a disease selected from NPC and ALS, said method comprising administering a therapy for treatment of said disease selected from NPC and ALS to said individual, wherein said individual is diagnosed with said disease selected from NPC and ALS by a method comprising the steps of:
a) providing a PBMC sample obtained from said individual,
b) detecting Hsp70 in said PBMC sample,
c) quantifying or determining the level of Hsp70 in said PBMC sample,
d) classifying or determining whether or not the individual has, or is likely to have a disease selected from NPC and ALS,
wherein said Hsp70 is HspA1A and HspA1B.

A therapy for treatment of a disease selected from NPC and ALS and a bioactive agent for same purpose are disclosed herein elsewhere and included in the above methods of treatment and therapies for disorders selected from NPC and ALS.

### Monitoring disease progression

The methods as disclosed herein are also useful for monitoring disease progression of a disease selected from NPC and ALS.

It is thus an aspect to provide a method for monitoring disease progression in an individual having a disease selected from NPC and ALS, said method comprising the steps of
a) providing one or more PBMC samples from said individual at two or more subsequent points in time,
b) detecting Hsp70 in each of said PBMC samples, and
c) quantifying or determining the level of Hsp70 in each of said PBMC samples,
wherein said Hsp70 is HspA1A and HspA1B.

In one embodiment detecting Hsp70 comprises detecting HspA1A and HspA1B.

In one embodiment the level of Hsp70 present in each of the PBMC samples is indicative of a progression of the disease or a remission of the disease.

It is understood that the steps of a) providing one or more PBMC samples from an individual, b) detecting Hsp70 in said PBMC samples, and c) quantifying or determining the level of Hsp70 in said PBMC samples, may each be performed according the present disclosure and specified herein elsewhere.

In one embodiment said method for monitoring disease progression comprises the further step of d) determining whether the disease selected from NPC and ALS is in progression or in remission.

In one embodiment said step d) comprise determining the level of Hsp70, wherein said Hsp70 is HspA1A and HspA1B, present in the PBMC sample at an earlier time, such as t=0, and determining the level of said Hsp70 present in the PBMC sample at a later time, such as t>0.

In one embodiment a first sample is taken at t=0 (earlier time) and one or more subsequent samples are taken at one or more later time points (later samples) at t>0. In one embodiment the PBMC samples are taken continuously at a certain interval.

In one embodiment the one or more subsequent samples comprise PBMC samples taken at t=0 and at 1, 2, 3, 4, 5, 6, 7, 8, 9, 10 or more time points after t=0.

In one embodiment the one or more subsequent samples are taken at one or more of t=1 day, t=2 days, t=3 days, t=4 days, t=5 days, t=6 days, t=7 days, t=14 days, t=3 weeks, t=4 weeks, t=5 weeks, t=6 weeks, t=7 weeks, t=8 weeks, t=1 month, t=2 months, t=3 months, t=4 months, t=5 months, t=6 months, t=7 months, t=8 months, t=9 months, t=10 months, t=11 months, t=12 months, t=13 months, t=14 months, t=15 months, t=16 months, t=17 months, t=18 months, t=20 months, t=22 months and/or t=24 months.

In one embodiment the one or more subsequent samples are taken at an interval of 1 day, 2 days, 3 days, 4 days, 5 days, 6 days, 7 days, 14 days, 3 weeks, 4 weeks, 5 weeks, 6 weeks, 7 weeks, 8 weeks, 1 month, 2 months, 3 months, 4 months, 5 months, 6 months, 7 months, 8 months, 9 months, 10 months, 11 months and/or 12 months. An interval of 1 month means that a subsequent sample is taken every 1 month,

In one embodiment a decrease in the level of Hsp70 over time is indicative of a progression of the disease.

In one embodiment an increase in the level of Hsp70 over time is indicative of a remission of the disease.

In one embodiment said method comprises determining whether the level of Hsp70 is lower in the subsequent sample(s), which is indicative of a progression of the disease; and/or determining whether the level of Hsp70 is higher in the subsequent sample(s), which is indicative of a remission of the disease.

In one embodiment a decrease in the level of Hsp70 over time measured at t=0 and at one or more time points at t>0 of 1 to 1000 times is indicative of a progression of the disease; such as a decrease of 1 to 2 times, 2 to 3 times, 3 to 4 times, 4 to 5 times, 5 to 6 times, 6 to 7 times, 7 to 8 times, 8 to 9 times, 9 to 10 times, 10 to 11 times, 11 to 12 times, 12 to 13 times, 13 to 14 times, 14 to 15 times, 15 to 16 times, 16 to 17 times, 17 to 18 times, 18 to 19 times, 19 to 20 times, 20 to 25 times, 25 to 30 times, 30 to 35 times, 35 to 40 times, 40 to 45 times, 45 to 50 times, 50 to 60 times, 60 to 70 times, 70 to 80 times, 80 to 90 times, 90 to 100 times, 100 to 150 times, 150 to 200 times, 200 to 250 times, 250 to 300 times, 300 to 400 times, 400 to 500 times, 500 to 600 times, 600 to 700 times, 700 to 800 times, such as a decrease in the level of Hsp70 of 900 to 1000 times.

In one embodiment an increase in the level of Hsp70 over time measured at t=0 and at one or more time points at t>0 of 1 to 1000 times is indicative of a remission of the disease; such as an increase of 1 to 2 times, 2 to 3 times, 3 to 4 times, 4 to 5 times, 5 to 6 times, 6 to 7 times, 7 to 8 times, 8 to 9 times, 9 to 10 times, 10 to 11 times, 11 to 12 times, 12 to 13 times, 13 to 14 times, 14 to 15 times, 15 to 16 times, 16 to 17 times, 17 to 18 times, 18 to 19 times, 19 to 20 times, 20 to 25 times, 25 to 30 times, 30 to 35 times, 35 to 40 times, 40 to 45 times, 45 to 50 times, 50 to 60 times, 60 to 70 times, 70 to 80 times, 80 to 90 times, 90 to 100 times, 100 to 150 times, 150 to 200 times, 200 to 250 times, 250 to 300 times, 300 to 400 times, 400 to 500 times, 500 to 600 times, 600 to 700 times, 700 to 800 times, such as an increase in the level of Hsp70 of 900 to 1000 times.

In one embodiment a decrease in the level of Hsp70 over time measured at t=0 and at t=6 months (approx.) of 500 to 20000 pg/mL PBMC is indicative of a progression of the disease, such as 500 to 750 pg/mL, such as 750 to 1000 pg/mL, such as 1000 to 1500 pg/mL, such as 1500 to 2000 pg/mL, such as 2000 to 3000 pg/mL, such as 3000 to 4000 pg/mL, such as 4000 to 5000 pg/mL, such as 5000 to 7500 pg/mL, such as 7500 to 10000 pg/mL, such as 10000 to 12500 pg/mL, such as 12500 to 15000 pg/mL, such as 15000 to 20000 pg/mL PBMC.

In one embodiment said method for monitoring disease progression in an individual having a disease selected from NPC and ALS comprises monitoring disease progression in an individual having a disease selected fromNPC and ALS.

### Monitoring efficacy of a therapy

The methods as disclosed herein are also useful for monitoring efficacy of a treatment or a potential treatment of a disease selected from NPC and ALS.

It is thus an aspect of the present disclosure to provide a method for monitoring efficacy of a therapy for treatment of a disease selected from NPC and ALS in an individual having a disease selected from NPC and ALS, said method comprising the steps of
a) providing one or more PBMC samples obtained from said individual before, during and/or after said therapy has been applied, maintained, reduced or elevated,
b) detecting Hsp70 in each of said one or more PBMC samples, and
c) quantifying or determining the level of Hsp70 in each of said one or more PBMC samples,
wherein said Hsp70 is HspA1A and HspA1B.

In one embodiment detecting Hsp70 comprises detecting HspA1A and HspA1B.

In one embodiment the level of Hsp70 present in each of the PBMC samples is indicative of efficacy of a therapy for a disease selected from NPC and ALS.

It is understood that the steps of a) providing one or more PBMC samples from an individual, b) detecting Hsp70 in said PBMC samples, and c) quantifying or determining the level of Hsp70 in said PBMC samples, may each be performed according the present disclosure and specified herein elsewhere.

In one embodiment said method for monitoring efficacy of a therapy for treatment of a disease selected from NPC and ALS comprises the further step of d) monitoring efficacy of a therapy for a disease selected from NPC and ALS.

In one embodiment said step d) comprise determining the level of Hsp70 present in a PBMC sample before a therapy has been applied, maintained, reduced or elevated.

In one embodiment said step d) comprise determining the level Hsp70 present in a PBMC sample during a therapy.

In one embodiment said step d) comprise determining the level of Hsp70 present in a PBMC sample after a therapy has been applied, maintained, reduced or elevated.

In one embodiment said step d) comprise one or more of i) determining the level of Hsp70 present in a PBMC sample before a therapy has been applied, maintained, reduced or elevated; ii) determining the level of Hsp70 present in the PBMC sample during a therapy, and iii) determining the level of Hsp70 present in the PBMC sample after a therapy has been applied, maintained, reduced or elevated.

In one embodiment an increase in the level of Hsp70 after a therapy has been applied, maintained, reduced or elevated, is indicative of the therapy being efficacious.

In one embodiment a decrease in the level of Hsp70 after a therapy has been applied, maintained, reduced or elevated, is indicative of the therapy being inefficacious.

In one embodiment said method comprises one or more steps of determining whether the level of Hsp70 is higher after a therapy has been applied, maintained, reduced or elevated, which is indicative of the therapy being efficacious; and/or one or more steps of determining whether the level of Hsp70 is lower after a therapy has been applied, maintained, reduced or elevated, which is indicative of the therapy being inefficacious.

In one embodiment an increase in the level of Hsp70 after a therapy has been applied, maintained, reduced or elevated, of 1 to 1000 times is indicative of the therapy being efficacious; such as an increase of 1 to 2 times, 2 to 3 times, 3 to 4 times, 4 to 5 times, 5 to 6 times, 6 to 7 times, 7 to 8 times, 8 to 9 times, 9 to 10 times, 10 to 11 times, 11 to 12 times, 12 to 13 times, 13 to 14 times, 14 to 15 times, 15 to 16 times, 16 to 17 times, 17 to 18 times, 18 to 19 times, 19 to 20 times, 20 to 25 times, 25 to 30 times, 30 to 35 times, 35 to 40 times, 40 to 45 times, 45 to 50 times, 50 to 60 times, 60 to 70 times, 70 to 80 times, 80 to 90 times, 90 to 100 times, 100 to 150 times, 150 to 200 times, 200 to 250 times, 250 to 300 times, 300 to 400 times, 400 to 500 times, 500 to 600 times, 600 to 700 times, 700 to 800 times, such as 900 to 1000 times

In one embodiment a decrease in the level of Hsp70 after a therapy has been applied, maintained, reduced or elevated, of 1 to 1000 times is indicative of the therapy being inefficacious; such as a decrease of 1 to 2 times, 2 to 3 times, 3 to 4 times, 4 to 5 times, 5 to 6 times, 6 to 7 times, 7 to 8 times, 8 to 9 times, 9 to 10 times, 10 to 11 times, 11 to 12 times, 12 to 13 times, 13 to 14 times, 14 to 15 times, 15 to 16 times, 16 to 17 times, 17 to 18 times, 18 to 19 times, 19 to 20 times, 20 to 25 times, 25 to 30 times, 30 to 35 times, 35 to 40 times, 40 to 45 times, 45 to 50 times, 50 to 60 times, 60 to 70 times, 70 to 80 times, 80 to 90 times, 90 to 100 times, 100 to 150 times, 150 to 200 times, 200 to 250 times, 250 to 300 times, 300 to 400 times, 400 to 500 times, 500 to 600 times, 600 to 700 times, 700 to 800 times, such as 900 to 1000 times.

In one embodiment said method for monitoring efficacy of a therapy for treatment of a disease selected from NPC and ALS in an individual having a disease selected from NPC and ALS, comprises monitoring efficacy of a therapy for treatment in an individual having a disease selected from NPC and ALS.

A therapy for treatment of a disease selected from NPC and ALS and a bioactive agent for same purpose are disclosed herein elsewhere and included in the above methods of monitoring efficacy of a therapy.

### Sample

The methods disclosed herein comprise the provision of a peripheral blood mononuclear cell (PBMC) sample from an individual.

It is understood that the samples of the present disclosure are obtained from or obtainable from an individual, preferably a human being. In one embodiment said PBMC sample is obtained from or obtainable from an individual.

A peripheral blood mononuclear cell (PBMC) is any peripheral blood cell having a round nucleus; these cells consist of lymphocytes (T cells, B cells, NK cells), monocytes and dendritic cells. In contrast erythrocytes and platelets have no nuclei, and granulocytes (neutrophils, basophils, and eosinophils) have multi-lobed nuclei.

Thus, in one embodiment a PBMC sample is a sample comprising peripheral blood mononuclear cells having a round nucleus. In one embodiment a PBMC sample is a sample comprising lymphocytes (T cells, B cells, NK cells), monocytes and/or dendritic cells.

A PBMC sample from an individual as defined herein is in one embodiment a sample that predominantly contains PBMCs. In one embodiment a PBMC sample is a sample comprising PBMCs extracted from whole blood. In one embodiment a PBMC sample is a sample comprising or consisting essentially of the PBMC component of whole blood.

PBMCs can be extracted from whole blood using ficoll, a hydrophilic polysaccharide that separates layers of blood, and gradient centrifugation, which will separate the blood into a top layer of plasma, followed by a layer of PBMCs and a bottom fraction of polymorphonuclear cells (such as neutrophils and eosinophils) and erythrocytes. The polymorphonuclear cells can be further isolated by lysing the red blood cells. Basophils are sometimes found in both the denser and the PBMC fractions.

When peripheral whole blood is drawn for human immune system studies, it is often processed to remove red blood cells by density gradient centrifugation. Most commonly this method uses Ficoll Paque, a solution of high molecular weight sucrose polymers. Ficoll separates whole blood into two fractions above and below the density of 1.077g/ml.

Peripheral blood mononuclear cells (PBMC) are the populations of immune cells that remain at the less dense, upper interface of the Ficoll layer, often referred to as the *buffy coat* and are the cells collected when the Ficoll fractionation method is used. Erythrocytes (red blood cells) and polymorphonuclear cells (PMNs) which include neutrophils and eosinophils are generally removed during this fractionation as they are denser than 1.077g/ml. Basophils, however can be greater or less dense then 1.077g/ml and thus may be present to a small degree in the less dense PBMC fraction.

The typical composition of PBMCs include lymphocytes (T cells, B cells, and NK cells) in the range of 70 - 90% of PBMCs, monocytes in the range of 10 - 30% of PBMCs, while dendritic cells are rare, being only 1 - 2% of PBMCs. The frequencies of cell types within the lymphocyte population in some embodiments may include 70 - 85% CD3+ T cells (45 - 70% of PBMC), 5 - 20% B cells (up to 15% of PBMC), and 5 - 20% NK cells (up to 15% of PBMC).

The CD3+ compartment is composed of CD4 (25 - 60% of PBMC) and CD8 T cells (5 - 30% of PBMC), in a roughly 2:1 ratio. Both CD4 and CD8 T cells can be further subset into the naïve, and the antigen-experienced central memory, effector memory, and effector subtypes that exist in resting or activated states. Multiple markers can be used to identify these compartments to varying similarities and thus the frequencies reported using different markers may vary.

CD4 T cells are known as helper T cells and can be further classified into various functional subtypes based on the expression profiles of specific cytokines, surface markers, or transcription factors. These include regulatory T cells, TH1, TH2, and TH17 cells as well as other described subpopulations such as TH9, follicular helper, and TR1 types. The cytotoxic CD8 T cell compartment has been to shown to be extremely heterogeneous in marker expression and function and may be comprised of roughly 200 functional phenotypes.

Circulating B cells include transitional, naïve, and memory subtypes as well as plasmablasts, all of which can be found at varying populations in peripheral blood. Circulating dendritic cells include plasmacytoid dendritic cells as well as myeloid derived dendritic cells. Circulating monocytes have been described as either being classical monocytes or nonclassical CD16+ proinflammatory monocytes, which comprise up to 10% of the monocytes in peripheral blood and have unique functions compared with classical monocytes.

In one embodiment the step a) of the methods disclosed herein of providing one or more PBMC samples from an individual comprise one or more steps of:
i) providing a whole blood sample, and
ii) separating whole blood into its subcomponents to obtain a PBMC sample.

In one embodiment said whole blood sample is subject to centrifugation and/or ficoll separation to obtain a PBMC sample.

### Sample from individual

The PBMC samples provided herein in one embodiment originates from an individual having, suspected of having, at risk of having or likely to have a disease selected from NPC and ALS.

In one embodiment said individual having, at risk of having, suspected of having or likely to have a disease selected from NPC and ALS is an individual having, at risk of having, suspected of having or likely to have NPC.

In one embodiment the PBMC sample is obtained from or obtainable from an individual having, suspected of having, at risk of having, or likely to have a disease selected from NPC and ALS.

In one embodiment the sample is obtained from an individual having, suspected of having, at risk of having, or likely to have a disease selected from NPC and ALS, as compared to the general population. An increased risk of having or developing a disease selected from NPC and ALS may be based on an assessment of family history, an assessment of symptoms and/or the result of other diagnostic tests for a disease selected from NPC and ALS.

In one embodiment the sample is obtained from an individual having one or more family members diagnosed with a disease selected from NPC and ALS.

In one embodiment the sample is obtained from an individual having a sibling, a parent, a cousin, an uncle and/or an aunt with a disease selected from NPC and ALS.

In one embodiment the sample is obtained from an individual having a sibling with a disease selected from NPC and ALS.

In one embodiment the sample is obtained from an individual predisposed for developing a disease selected from NPC and ALS.

In one embodiment the sample is obtained from an individual having one or more family members with a genetic predisposition for a disease selected from NPC and ALS.

As specified herein elsewhere 'a disease presenting with a reduced level of Hsp70' in one embodiment includes a disease selected from NPC and ALS.

In one embodiment the sample is obtained from an individual having one or more family members with a mutation in the *NPC1* gene and/or the *NPC2* gene.

In one embodiment the sample is obtained from an individual with one or more symptoms associated with a disease selected from NPC and ALS; such as one or more symptoms indicative of a disease selected from NPC and ALS.

In one embodiment the sample is obtained from an individual with one or more symptoms associated with the LSD NPC; such as one or more symptoms indicative of the LSD NPC.

Although the signs and symptoms vary from disease to disease, symptoms of LSD occur in each case because of a protein deficiency that inhibits the ability of the lysosomes present in each of the body's cells to perform their normal function.

In one embodiment the sample is obtained from an individual with one or more symptoms associated with Niemann Pick disease Type C; in one embodiment the symptoms are selected from the group consisting of vertical gaze palsy, enlarged liver, enlarged spleen and/or jaundice, progressive loss of motor skills, feeding difficulties, progressive learning disabilities, and seizures.

Other symptoms of LSD and NPC include deterioration both intellect and neurological functions, progressive vision failure (optic atrophy), neurological disturbances, mental deterioration, enlarged liver and/or spleen (hepatosplenomegaly), physical deterioration, progressive muscle weakness, diminished muscle tone (hypotonia), motor delays, feeding problems, respiratory difficulties and general weakness (lethargy).

### Diseases presenting with a reduced level of Hsp70

Hsp70 serves several important roles in cell homeostasis and a number of diseases present with a reduced level of Hsp70, including lysosomal storage diseases, neurodegenerative diseases, and some neuromuscular and muscular diseases.

In one embodiment, a disease as referred to herein throughout is selected from NPC and ALS.

### Lysosomal Storage Disease

One aspect of the present disclosure relates to detecting Hsp70 in PBMC samples obtained from or obtainable from an individual having, suspected of having, at risk of having, or likely to have the lysosomal storage disease NPC; methods of diagnosing the lysosomal storage disease NPC in an individual; methods for monitoring disease progression in an individual having the lysosomal storage disease NPC; and methods for monitoring efficacy of a therapy for treatment of the lysosomal storage disease NPC in an individual having NPC.

Reference to a lysosomal storage disease in the present context is meant to encompass each and any lysosomal storage disease known to the skilled person.

Lysosomal storage diseases include lipid storage disorders (or lipidosis) including the sphingolipidoses; mucopolysaccharidoses; glycogen storage disorders; disorders of glycoprotein metabolism (glycoproteinosis); and mucolipidoses.

In one embodiment of the present disclosure there is provided methods of diagnosing a lysosomal storage disease in an individual, as outlined in detail herein above, wherein said LSD is NPC.

In one embodiment of the present disclosure there is provided methods for monitoring disease progression in an individual having a lysosomal storage disease, as outlined in detail herein above, wherein said LSD is NPC.

In one embodiment of the present disclosure there is provided methods for monitoring efficacy of a therapy for treatment of a lysosomal storage disease in an individual having a lysosomal storage disease, as outlined in detail herein above, wherein said LSD NPC.

Sphingolipidoses are a heterogeneous group of inherited disorders of sphingolipid metabolism affecting primarily the central nervous system. These disorders occur chiefly in the pediatric population, and the degenerative nature of the disease processes is generally characterized by diffuse and progressive involvement of neurons (gray matter) with psychomotor retardation and myoclonus or of fiber tracts (white matter) with weakness and spasticity. The accumulated sphingolipid include gangliosides (the gangliosidoses), glycolipids/ceramide (Fabry disease, Krabbe disease), glucocerebrosides (Gaucher disease), sphingomyelin (Niemann Pick disease) and sulfatide (leukodystrohies; MLD).

In one embodiment a lysosomal storage disease as disclosed herein is Niemann Pick disease Type C.

### Neurodegenerative diseases

One aspect of the present disclosure relates to methods of detecting Hsp70 in PBMC samples obtained from or obtainable from an individual having, suspected of having, at risk of having, or likely to have the neurodegenerative disease ALS.

Neurodegenerative diseases are a growing cause of disability in the aging community. Neurodegeneration, the slow progression of dysfunction associated with a loss of neurons and axonal connections in the central nervous system (CNS), is the primary pathological characteristic of such neurological disorders as Alzheimer's disease, Parkinson's disease (PD) and Huntington's disease (HD). This loss results in gross atrophy of the affected regions, including degeneration in the temporal lobe and parietal lobe, and parts of the frontal cortex and cingulate gyrus.

Many neurodegenerative diseases are caused by genetic mutations, most of which are located in completely unrelated genes. In many of the different diseases, the mutated gene has a common feature: a repeat of the CAG nucleotide triplet (encodes glutamine). A repeat of CAG results in a polyglutamine (polyQ) tract, and diseases showing this are known as polyglutamine diseases (polyQ diseases). These include Huntington's disease, spinocerebellar ataxias, DRPLA (Dentatorubropallidoluysian atrophy) and SBMA (Spinobulbar muscular atrophy or Kennedy disease).

In one embodiment a neurodegenerative disorder as disclosed herein is Amyotrophic lateral sclerosis (ALS; Lou Gehrig's Disease).

### Muscular diseases

One aspect of the present disclosure relates to methods of detecting Hsp70 in PBMC samples obtained from or obtainable from an individual having, suspected of having, at risk of having, or likely to have a muscular disease, such as a muscular disease selected from the group consisting of a neuromuscular disorder, muscular dystrophy and an inflammatory muscle disorder, wherein said disease is ALS.

### Neuromuscular disorders

Neuromuscular disorders affect the nerves that control your voluntary muscles. Voluntary muscles are the ones you can control, like in your arms and legs. Your nerve cells, also called neurons, send the messages that control these muscles. When the neurons become unhealthy or die, communication between your nervous system and muscles breaks down. As a result, your muscles weaken and waste away. The weakness can lead to twitching, cramps, aches and pains, and joint and movement problems. Sometimes it also affects heart function and your ability to breathe. Some examples of central neuromuscular disorders include cerebrovascular accident, Parkinson's disease, multiple sclerosis, Huntington's disease and Creutzfeldt-Jakob disease. Spinal muscular atrophies are disorders of lower motor neuron while amyotrophic lateral sclerosis is a mixed upper and lower motor neuron condition.

In one embodiment the neuromuscular disorder is Amyotrophic lateral sclerosis (ALS).

### Muscular dystrophies

Muscular dystrophy (MD) is a group of muscle diseases that results in increasing weakening and breakdown of skeletal muscles over time. The disorders differ in which muscles are primarily affected, the degree of weakness, how fast they worsen, and when symptoms begin. Many people will eventually become unable to walk. Some types are also associated with problems in other organs. There are nine main categories of muscular dystrophy that contain more than thirty specific types.

### Therapies for treatment

According to the present disclosure there is provided methods of administering a therapy for treatment of a disease selected from NPC and ALS to a patient diagnosed with a disease selected from NPC and ALS.

In one embodiment step e) of administering a therapy for treatment of a disease selected from NPC and ALS to a patient diagnosed with said disease selected from NPC and ALS comprises administering an effective amount of a bioactive agent to said individual.

A therapy for treatment of a disease selected from NPC and ALS and a bioactive agent for same purpose includes known therapies for disease selected from NPC and ALS.

A "Bioactive agent" (i. e., biologically active substance/agent) is any agent, drug, substance, compound, composition of matter or mixture which provides some pharmacologic, often beneficial, effect that can be demonstrated *in vivo* or *in vitro.* As used herein, this term further includes any physiologically or pharmacologically active substance that produces a localized or systemic effect in an individual.

### Hsp70 and Hsp inducers

In one embodiment step e) of administering a therapy for treatment of a disease selected from NPC and ALS to a patient diagnosed with said disease selected from NPC and ALS comprises administering an effective amount of a bioactive agent that increase the intracellular concentration and/or activity of heat shock proteins, such as Hsp70.

In one embodiment a therapy for treatment of a disease selected from NPC and ALS is a bioactive agent that increases the intracellular concentration (or levels) and/or activity of one or more heat shock proteins, including Hsp70 and co-chaperones; and includes Hsp70 itself, or a functional fragment or variant thereof, any heat shock protein inducer and any Hsp70 inducer known to the skilled person.

A bioactive agent that increases the intracellular concentration and/or activity of one or more heat shock proteins, including Hsp70, and a bioactive agent that increases the intracellular concentration and/or activity of Hsp70, can be used interchangeably with 'Hsp70 inducer' herein.

An Hsp70 inducer can amplify Hsp70 gene expression and protein expression with or without a concomitant stress. A direct Hsp70 inducer is a compound that can by itself amplify Hsp70 gene expression and protein expression without a concomitant stress. An indirect Hsp70 inducer, or an Hsp70 co-inducer, is a compound that cannot amplify Hsp70 gene expression and protein expression without a concomitant (mild) stress, but the stress-induced increase in Hsp70 levels is further elevated or enhanced by their presence.

It follows that a bioactive agent may increase the intracellular concentration and/or activity of heat shock proteins, such as Hsp70, either directly or indirectly.

In one embodiment, the bioactive agent is Hsp70 protein, or a functional fragment or variant thereof. In one embodiment said Hsp70 protein is selected from HSPA1A and HSPA1B, or a functional fragment or variant thereof. In one embodiment said functional fragment or variant of Hsp70 has at least 75% sequence identity to Hsp70 such as to any one of HSPA1A and HSPA1B, such as at least 80%, at least 85%, at least 90%, at least 95% or at least 99% sequence identity.

In another embodiment, the bioactive agent is an inducer of heat shock proteins, including Hsp70.

In one embodiment the inducer of heat shock proteins, including Hsp70, is an inducer of one or more of Hsp70, Hsp40, Hsp72 and Hsp90, and co-chaperones.

In one embodiment the inducer of heat shock proteins is an inducer of at least Hsp70. In one embodiment the inducer of heat shock proteins is an inducer of Hsp70.

Reference to an inducer of Hsp70, or inducing Hsp70, implies that at least Hsp70 is induced, and does not exclude co-induction of other proteins and effectors such as other heat shock proteins. An inducer of Hsp70 refers equally to Hsp70 inducers and co-inducers, and direct and indirect Hsp70 inducers.

In one embodiment, the bioactive agent comprises a combination of Hsp70, or a functional fragment or variant thereof, and an inducer of heat shock proteins including Hsp70.

In one embodiment the bioactive agent activates the heat shock response. In one embodiment the bioactive agent increases the intracellular concentration and/or activity of one or more heat shock proteins, including Hsp70. In one embodiment the bioactive agent increases the intracellular concentration (or level) and/or activity of Hsp70. In one embodiment the bioactive agent increases the intracellular concentration (or level) of Hsp70. In one embodiment the bioactive agent is an inducer of one or more heat shock proteins, including Hsp70. In one embodiment the bioactive agent is an inducer of Hsp70.

### Small molecule inducers of heat shock proteins

In one embodiment the bioactive agent is a small molecule inducer of heat shock proteins, including Hsp70, such as a small molecule inducer of Hsp70.

In one embodiment a small molecule inducer of one or more heat shock proteins, including Hsp70; is a compound capable of increasing the intracellular concentration (or level) of *inter alia* Hsp70, such as by amplifying Hsp70 gene expression.

In one embodiment the bioactive agent is capable of increasing the intracellular concentration (or levels) of Hsp70 by amplifying Hsp70 gene expression. In one embodiment the bioactive agent is capable of increasing the intracellular concentration (or level) of Hsp70 by amplifying Hsp70 gene expression, wherein said bioactive agent is a hydroxylamine derivative, such as a hydroxylamine derivative small molecule.

Examples of such hydroxylamine derivatives include arimoclomol, iroxanadine, bimoclomol, BGP-15, their stereoisomers and the acid addition salts thereof.

### Arimoclomol:

In one embodiment the small molecule inducer of Hsp70 is selected from N-[2-hydroxy-3-(1-piperidinyl)-propoxy]-pyridine-1-oxide-3-carboximidoyl chloride (arimoclomol), its stereoisomers and the acid addition salts thereof. Arimoclomol is further described in e.g. WO 00/50403.

In one embodiment the small molecule inducer of Hsp70 is selected from N-[2-hydroxy-3-(1-piperidinyl)-propoxy]-pyridine-1-oxide-3-carboximidoyl chloride (arimoclomol), its optically active (+) or (-) enantiomer, a mixture of the enantiomers of any ratio, and the racemic compound, furthermore, the acid addition salts formed from any of the above compounds with mineral or organic acids constitute objects of the present disclosure. All possible geometrical isomer forms of N-[2-hydroxy-3-(1-piperidinyl)-propoxy]-pyridine-1-oxide-3-carboximidoyl chloride belong to the scope of the disclosure. The term "the stereoisomers of N-[2-hydroxy-3-(1-piperidinyl)-propoxy]-pyridine-1-oxide-3-carboximidoyl chloride" refers to all possible optical and geometrical isomers of the compound.

If desired, the N-[2-hydroxy-3-(1-piperidinyl)-propoxy]-pyridine-1-oxide-3-carboximidoyl chloride or one of its optically active enantiomers can be transformed into an acid addition salt with a mineral or organic acid, by known methods.

In one embodiment the small molecule inducer of Hsp70 is the racemate of N-[2-hydroxy-3-(1-piperidinyl)-propoxy]-pyridine-1-oxide-3-carboximidoyl chloride.

In one embodiment the small molecule inducer of Hsp70 is an optically active stereoisomer of N-[2-hydroxy-3-(1-piperidinyl)-propoxy]-pyridine-1-oxide-3-carboximidoyl chloride.

In one embodiment the small molecule inducer of Hsp70 is an enantiomer of N-[2-hydroxy-3-(1-piperidinyl)-propoxy]-pyridine-1-oxide-3-carboximidoyl chloride.

In one embodiment the small molecule inducer of Hsp70 is selected from the group consisting of (+)-R-N-[2-hydroxy-3-(1-piperidinyl)-propoxy]-pyridine-1-oxide-3-carboximidoyl chloride and (-)-(S)-N-[2-hydroxy-3-(1-piperidinyl)-propoxy]-pyridine-1-oxide-3-carboximidoyl chloride.

In one embodiment the small molecule inducer of Hsp70 is an acid addition salt of N-[2-hydroxy-3-(1-piperidinyl)-propoxy]-pyridine-1-oxide-3-carboximidoyl chloride.

In one embodiment the small molecule inducer of Hsp70 is selected from the group consisting of N-[2-hydroxy-3-(1-piperidinyl)-propoxy]-pyridine-1-oxide-3-carboximidoyl chloride citrate (BRX-345), and N-[2-hydroxy-3-(1-piperidinyl)-propoxy]-pyridine-1-oxide-3-carboximidoyl chloride maleate (BRX-220).

In one embodiment the small molecule inducer of Hsp70 is selected from the group consisting of (+)-R-N-[2-hydroxy-3-(1-piperidinyl)-propoxy]-pyridine-1-oxide-3-carboximidoyl chloride citrate; (-)-S-N-[2-hydroxy-3-(1-piperidinyl)-propoxy]-pyridine-1-oxide-3-carboximidoyl chloride citrate; (+)-R-N-[2-hydroxy-3-(1-piperidinyl)-propoxy]-pyridine-1-oxide-3-carboximidoyl chloride maleate; and (-)-S-N-[2-hydroxy-3-(1-piperidinyl)-propoxy]-pyridine-1-oxide-3-carboximidoyl chloride maleate.

### BGP-15:

In one embodiment the small molecule inducer of Hsp70 is N-[2-hydroxy-3-(1-piperidinyl) propoxy]-3-pyridinecarboximidamide, dihydrochloride (BGP-15), its stereoisomers and the acid addition salts thereof.

In one embodiment the small molecule inducer of Hsp70 is selected from N-[2-hydroxy-3-(1-piperidinyl)propoxy]-3-pyridinecarboximidamide, dihydrochloride (BGP-15), its optically active (+) or (-) enantiomer, a mixture of the enantiomers of any ratio, and the racemic compound, furthermore, the acid addition salts formed from any of the above compounds with mineral or organic acids constitute objects of the present disclosure. All possible geometrical isomer forms of N-[2-hydroxy-3-(1-piperidinyl)propoxy]-3-pyridinecarboximidamide, dihydrochloride belong to the scope of the disclosure. The term "the stereoisomers of N-[2-hydroxy-3-(1-piperidinyl)propoxy]-3-pyridine-carboximidamide, dihydrochloride" refers to all possible optical and geometrical isomers of the compound.

### Iroxanadine:

In one embodiment the small molecule inducer of Hsp70 is selected from 5,6-dihydro-5-(1-piperidinyl)methyl-3-(3-pyridyl)-4H-1,2,4-oxadiazine (iroxanadine), its stereoisomers and the acid addition salts thereof. Iroxanadine is further described in e.g. WO 97/16439 and WO 00/35914.

In one embodiment the small molecule inducer of Hsp70 is selected from 5,6-dihydro-5-(1-piperidinyl)methyl-3-(3-pyridyl)-4H-1,2,4-oxadiazine (iroxanadine), its optically active (+) or (-) enantiomer, a mixture of the enantiomers of any ratio, and the racemic compound, furthermore, the acid addition salts formed from any of the above compounds with mineral or organic acids constitute objects of the present disclosure. All possible geometrical isomer forms of 5,6-dihydro-5-(1-piperidinyl)methyl-3-(3-pyridyl)-4H-1,2,4-oxadiazine belong to the scope of the disclosure. The term "the stereoisomers of 5,6-dihydro-5-(1-piperidinyl)methyl-3-(3-pyridyl)-4H-1,2,4-oxadiazine" refers to all possible optical and geometrical isomers of the compound.

If desired, the 5,6-dihydro-5-(1-piperidinyl)methyl-3-(3-pyridyl)-4H-1,2,4-oxadiazine or one of its optically active enantiomers can be transformed into an acid addition salt with a mineral or organic acid, by known methods.

In one embodiment the small molecule inducer of Hsp70 is the racemate of 5,6-dihydro-5-(1-piperidinyl)methyl-3-(3-pyridyl)-4H-1,2,4-oxadiazine.

In one embodiment the small molecule inducer of Hsp70 is an optically active stereoisomer of 5,6-dihydro-5-(1-piperidinyl)methyl-3-(3-pyridyl)-4H-1,2,4-oxadiazine.

In one embodiment the small molecule inducer of Hsp70 is an enantiomer of 5,6-dihydro-5-(1-piperidinyl)methyl-3-(3-pyridyl)-4H-1,2,4-oxadiazine.

In one embodiment the small molecule inducer of Hsp70 is selected from the group consisting of (+)-5,6-dihydro-5-(1-piperidinyl)methyl-3-(3-pyridyl)-4H-1,2,4-oxadiazine and (-)-5,6-dihydro-5-(1-piperidinyl)methyl-3-(3-pyridyl)-4H-1,2,4-oxadiazine.

In one embodiment the small molecule inducer of Hsp70 is an acid addition salt of 5,6-dihydro-5-(1-piperidinyl)methyl-3-(3-pyridyl)-4H-1,2,4-oxadiazine.

In one embodiment the small molecule inducer of Hsp70 is selected from the group consisting of 5,6-dihydro-5-(1-piperidinyl)methyl-3-(3-pyridyl)-4H-1,2,4-oxadiazine citrate, and 5,6-dihydro-5-(1-piperidinyl)methyl-3-(3-pyridyl)-4H-1,2,4-oxadiazine maleate.

In one embodiment the small molecule inducer of Hsp70 is selected from the group consisting of (+)-5,6-dihydro-5-(1-piperidinyl)methyl-3-(3-pyridyl)-4H-1,2,4-oxadiazine citrate; (-)-5,6-dihydro-5-(1-piperidinyl)methyl-3-(3-pyridyl)-4H-1,2,4-oxadiazine citrate; (+)-5,6-dihydro-5-(1-piperidinyl)methyl-3-(3-pyridyl)-4H-1,2,4-oxadiazine maleate; and (-)-5,6-dihydro-5-(1-piperidinyl)methyl-3-(3-pyridyl)-4H-1,2,4-oxadiazine maleate.

### Bimoclomol:

In one embodiment the small molecule inducer of Hsp70 is selected from N-[2-hydroxy-3-(1-piperidinyl)-propoxy]-3-pyridinecarboximidoyl chloride (bimoclomol) its stereoisomers and the acid addition salts thereof. Bimoclomol is further described in e.g. WO 1997/16439.

In one embodiment the small molecule inducer of Hsp70 is selected from N-[2-hydroxy-3-(1-piperidinyl)-propoxy]-3-pyridinecarboximidoyl chloride (bimoclomol), its optically active (+) or (-) enantiomer, a mixture of the enantiomers of any ratio, and the racemic compound, furthermore, the acid addition salts formed from any of the above compounds with mineral or organic acids constitute objects of the present disclosure. All possible geometrical isomer forms of N-[2-hydroxy-3-(1-piperidinyl)-propoxy]-3-pyridinecarboximidoyl chloride belong to the scope of the disclosure. The term "the stereoisomers of N-[2-hydroxy-3-(1-piperidinyl)-propoxy]-3-pyridinecarboximidoyl chloride" refers to all possible optical and geometrical isomers of the compound.

If desired, the N-[2-hydroxy-3-(1-piperidinyl)-propoxy]-3-pyridinecarboximidoyl chloride or one of its optically active enantiomers can be transformed into an acid addition salt with a mineral or organic acid, by known methods.

In one embodiment the small molecule inducer of Hsp70 is the racemate of N-[2-hydroxy-3-(1-piperidinyl)-propoxy]-3-pyridinecarboximidoyl chloride.

In one embodiment the small molecule inducer of Hsp70 is an optically active stereoisomer of N-[2-hydroxy-3-(1-piperidinyl)-propoxy]-3-pyridinecarboximidoyl chloride.

In one embodiment the small molecule inducer of Hsp70 is an enantiomer of N-[2-hydroxy-3-(1-piperidinyl)-propoxy]-3-pyridinecarboximidoyl chloride.

In one embodiment the small molecule inducer of Hsp70 is selected from the group consisting of (+)-R-N-[2-hydroxy-3-(1-piperidinyl)-propoxy]-3-pyridinecarboximidoyl chloride and (-)-(S)-N-[2-hydroxy-3-(1-piperidinyl)-propoxy]-3-pyridinecarboximidoyl chloride.

In one embodiment the small molecule inducer of Hsp70 is an acid addition salt of N-[2-hydroxy-3-(1-piperidinyl)-propoxy]-3-pyridinecarboximidoyl chloride.

In one embodiment the small molecule inducer of Hsp70 is selected from the group consisting of N-[2-hydroxy-3-(1-piperidinyl)-propoxy]-3-pyridinecarboximidoyl chloride citrate, and N-[2-hydroxy-3-(1-piperidinyl)-propoxy]-3-pyridinecarboximidoyl chloride maleate.

In one embodiment the small molecule inducer of Hsp70 is selected from the group consisting of (+)-R-N-[2-hydroxy-3-(1-piperidinyl)-propoxy]-3-pyridinecarboximidoyl chloride citrate; (-)-S-N-[2-hydroxy-3-(1-piperidinyl)-propoxy]-3-pyridinecarboximidoyl chloride citrate; (+)-R-N-[2-hydroxy-3-(1-piperidinyl)-propoxy]-3-pyridinecarboximidoyl chloride maleate; and (-)-S-N-[2-hydroxy-3-(1-piperidinyl)-propoxy]-3-pyridine-carboximidoyl chloride maleate.

A number of compounds have been shown to induce (or co-induce) HSPs, including Hsp70. In one embodiment the inducer of Hsp70 is selected from the group consisting of: membrane-interactive compounds such as alkyllysophospholipid edelfosine (ET-18-OCH3 or 1-octadecyl-2-methyl-rac-glycero-3-phosphocholine); anti-inflammatory drugs including cyclooxygenase 1/2 inhibitors such as celecoxib and rofecoxib, as well as NSAIDs such as acetyl-salicylic acid, sodium salicylate and indomethacin; dexamethasone; prostaglandins PGA1, PGj2 and 2-cyclopentene-1-one; peroxidase proliferator-activated receptor-gamma agonists; tubulin-interacting anticancer agents including vincristine and paclitaxel; the insulin sensitizer pioglitazone; anti-neoplastic agents such as carboplatin, doxorubicin, fludarabine, ifosfamide and cytarabine; Hsp90 inhibitors including geldanamycin, 17-AAG, 17-DMAG, radicicol, herbimycin-A and arachidonic acid; proteasome inhibitors such as MG132, lactacystin, Bortezomib, Carfilzomib and Oprozomib; serine protease inhibitors such as DCIC, TLCK and TPCK; Histone Deacetylase Inhibitors (HDACi) including SAHA/vorinostat, Belinostat/PXD101, LB-205, LBH589 (panobinostat), FK-228, CI-994, trichostatin A (TSA) and PCI-34051; anti-ulcer drugs including geranylgeranylacetone (GGA), rebamipide, carbenoxolone and polaprezinc (zinc L-carnosine); heavy metals (zinc and tin); cocaine; nicotine; alcohol; alpha-adrenergic agonists; cyclopentenone prostanoids; L-type Ca++ channel blockers, such as L-type Ca++ channel blockers that also inhibits ryanodine receptors, such as lacidipine; ryanodine receptor antagonists such as DHBP (1,1'-diheptyl- 4,4'-bipyridium; as well as herbal medicines including paeoniflorin, glycyrrhizin, celastrol, dihydrocelastrol, dihydrocelastrol diacetate and curcumin.

In one embodiment the inducer of Hsp70 is a proteasome inhibitor. In one embodiment the proteasome inhibitor is selected from the group consisting of Bortezomib, Carfilzomib, Oprozomib, MG132 and lactacystin.

In one embodiment the inducer of Hsp70 is a HDAC inhibitor. In one embodiment the HDACi is selected form the group consisting of SAHA/vorinostat, Belinostat/PXD101, LB-205, LBH589 (panobinostat), FK-228, CI-994, trichostatin A (TSA) and PCI-34051.

In one embodiment the inducer of Hsp70 is a membrane fluidizer. Treatment with a membrane fluidizer may also be termed lipid therapy. In one embodiment the inducer of Hsp70 is a membrane fluidizer selected from the group consisting of benzyl alcohol, heptanol, AL721, docosahexaenoic acid, aliphatic alcohols, oleyl alcohol, dimethylaminoethanol, A₂C, farnesol and anaesthetics such as lidocaine, ropivacaine, bupivacaine and mepivacaine, as well as others known to the skilled person.

### Other treatments of LSD

In addition to the above described treatments with Hsp or Hsp inducing agents, LSD may be treated by other means as described herein below. Such treatments may be combined with the treatment with Hsp and/or Hsp inducers.

The underlying cause of LSDs is the inability of specific lysosomal enzymes to catabolize efficiently specific lysosomal substances such as lipids. Therefore the use of enzyme replacement therapy (ERT), by providing to a patient the recombinant enzyme, has been employed for a subset of these diseases, including Gaucher and Fabry disease. ERT is effective only towards the specific type of disease to which the recombinant enzyme has been produced.

In one embodiment step e) of administering a therapy for treatment of the lysosomal storage disease NPC to a patient diagnosed with said lysosomal storage disease comprises administering an effective amount of an enzyme replacement therapy (ERT).

In one embodiment step e) of administering a therapy for treatment of the lysosomal storage disease NPC to a patient diagnosed with said lysosomal storage disease comprises administering a substrate reduction therapy (SRT).

In one embodiment step e) of administering a therapy for treatment of the lysosomal storage disease NPC to a patient diagnosed with said lysosomal storage disease comprises administering a therapy selected from the group consisting of pain relievers; corticosteroids; a transplantation, such as bone marrow transplantation, cord blood transplantation or stem cell transplantation; and symptomatic and supportive therapy, such as physical therapy.

In one embodiment the therapy for treatment of Niemann Pick disease type C is selected from the group consisting of a small molecule inducer of heat shock proteins including Hsp70, such as arimoclomol; Ambroxol and Miglustat.

### Sequences

SEQ ID NO: 1: The protein sequence for Homo sapiens heat shock 70 kDa protein 1A (HSPA1A_HUMAN) (NM_005345.5 / UniProtKB - P0DMV8): 
MAKAAAIGIDLGTTYSCVGVFQHGKVEIIANDQGNRTTPSYVAFTDTERLIGDAAKNQVALNPQNTVFDA
KRLIGRKFGDPVVQSDMKHWPFQVINDGDKPKVQVSYKGETKAFYPEEISSMVLTKMKEIAEAYLGYPVT
NAVITVPAYFNDSQRQATKDAGVIAGLNVLRIINEPTAAAIAYGLDRTGKGERNVLIFDLGGGTFDVSIL
TIDDGIFEVKATAGDTHLGGEDFDNRLVNHFVEEFKRKHKKDISQNKRAVRRLRTACERAKRTLSSSTQA
SLEIDSLFEGIDFYTSITRARFEELCSDLFRSTLEPVEKALRDAKLDKAQIHDLVLVGGSTRIPKVQKLL
QDFFNGRDLNKSINPDEAVAYGAAVQAAILMGDKSENVQDLLLLDVAPLSLGLETAGGVMTALIKRNSTI
PTKQTQIFTTYSDNQPGVLIQVYEGERAMTKDNNLLGRFELSGIPPAPRGVPQIEVTFDIDANGILNVTA
TDKSTGKANKITITNDKGRLSKEEIERMVQEAEKYKAEDEVQRERVSAKNALESYAFNMKSAVEDEGLKG
KISEADKKKVLDKCQEVISWLDANTLAEKDEFEHKRKELEQVCNPIISGLYQGAGGPGPGGFGAQGPKGG
SGSGPTIEEVD
SEQ ID NO: 2: The initiator methionine (M at position 1) of SEQ ID NO:1 is removed to yield a 640-amino acid long sequence (position 2-641): 
AKAAAIGIDLGTTYSCVGVFQHGKVEIIANDQGNRTTPSYVAFTDTERLIGDAAKNQVALNPQNTVFDAK
RLIGRKFGDPVVQSDMKHWPFQVINDGDKPKVQVSYKGETKAFYPEEISSMVLTKMKEIAEAYLGYPVTN
AVITVPAYFNDSQRQATKDAGVIAGLNVLRIINEPTAAAIAYGLDRTGKGERNVLIFDLGGGTFDVSILT
IDDGIFEVKATAGDTHLGGEDFDNRLVNHFVEEFKRKHKKDISQNKRAVRRLRTACERAKRTLSSSTQAS
LEIDSLFEGIDFYTSITRARFEELCSDLFRSTLEPVEKALRDAKLDKAQIHDLVLVGGSTRIPKVQKLLQ
DFFNGRDLNKSINPDEAVAYGAAVQAAILMGDKSENVQDLLLLDVAPLSLGLETAGGVMTALIKRNSTIP
TKQTQIFTTYSDNQPGVLIQVYEGERAMTKDNNLLGRFELSGIPPAPRGVPQIEVTFDIDANGILNVTAT
DKSTGKANKITITNDKGRLSKEEIERMVQEAEKYKAEDEVQRERVSAKNALESYAFNMKSAVEDEGLKGK
ISEADKKKVLDKCQEVISWLDANTLAEKDEFEHKRKELEQVCNPIISGLYQGAGGPGPGGFGAQGPKGGS
GSGPTIEEVD
SEQ ID NO: 3: The nucleic acid (DNA) sequence for Homo sapiens heat shock 70 kDa protein 1A (HSPA1A) (NM_005345.5): 
1 ataaaagccc aggggcaagc ggtccggata acggctagcc tgaggagctg ctgcgacagt
61 ccactacctt tttcgagagt gactcccgtt gtcccaaggc ttcccagagc gaacctgtgc
121 ggctgcaggc accggcgcgt cgagtttccg gcgtccggaa ggaccgagct cttctcgcgg
181 atccagtgtt ccgtttccag cccccaatct cagagcggag ccgacagaga gcagggaacc
241 ggcatggcca aagccgcggc gatcggcatc gacctgggca ccacctactc ctgcgtgggg
301 gtgttccaac acggcaaggt ggagatcatc gccaacgacc agggcaaccg caccaccccc
361 agctacgtgg ccttcacgga caccgagcgg ctcatcgggg atgcggccaa gaaccaggtg
421 gcgctgaacc cgcagaacac cgtgtttgac gcgaagcggc tgattggccg caagttcggc
481 gacccggtgg tgcagtcgga catgaagcac tggcctttcc aggtgatcaa cgacggagac
 
541 aagcccaagg tgcaggtgag ctacaagggg gagaccaagg cattctaccc cgaggagatc
601 tcgtccatgg tgctgaccaa gatgaaggag atcgccgagg cgtacctggg ctacccggtg
661 accaacgcgg tgatcaccgt gccggcctac ttcaacgact cgcagcgcca ggccaccaag
721 gatgcgggtg tgatcgcggg gctcaacgtg ctgcggatca tcaacgagcc cacggccgcc
781 gccatcgcct acggcctgga cagaacgggc aagggggagc gcaacgtgct catctttgac
841 ctgggcgggg gcaccttcga cgtgtccatc ctgacgatcg acgacggcat cttcgaggtg
901 aaggccacgg ccggggacac ccacctgggt ggggaggact ttgacaacag gctggtgaac
961 cacttcgtgg aggagttcaa gagaaaacac aagaaggaca tcagccagaa caagcgagcc
1021 gtgaggcggc tgcgcaccgc ctgcgagagg gccaagagga ccctgtcgtc cagcacccag
1081 gccagcctgg agatcgactc cctgtttgag ggcatcgact tctacacgtc catcaccagg
1141 gcgaggttcg aggagctgtg ctccgacctg ttccgaagca ccctggagcc cgtggagaag
1201 gctctgcgcg acgccaagct ggacaaggcc cagattcacg acctggtcct ggtcgggggc
1261 tccacccgca tccccaaggt gcagaagctg ctgcaggact tcttcaacgg gcgcgacctg
1321 aacaagagca tcaaccccga cgaggctgtg gcctacgggg cggcggtgca ggcggccatc
1381 ctgatggggg acaagtccga gaacgtgcag gacctgctgc tgctggacgt ggctcccctg
1441 tcgctggggc tggagacggc cggaggcgtg atgactgccc tgatcaagcg caactccacc
1501 atccccacca agcagacgca gatcttcacc acctactccg acaaccaacc cggggtgctg
1561 atccaggtgt acgagggcga gagggccatg acgaaagaca acaatctgtt ggggcgcttc
1621 gagctgagcg gcatccctcc ggcccccagg ggcgtgcccc agatcgaggt gaccttcgac
1681 atcgatgcca acggcatcct gaacgtcacg gccacggaca agagcaccgg caaggccaac
1741 aagatcacca tcaccaacga caagggccgc ctgagcaagg aggagatcga gcgcatggtg
1801 caggaggcgg agaagtacaa agcggaggac gaggtgcagc gcgagagggt gtcagccaag
1861 aacgccctgg agtcctacgc cttcaacatg aagagcgccg tggaggatga ggggctcaag
1921 ggcaagatca gcgaggcgga caagaagaag gtgctggaca agtgtcaaga ggtcatctcg
1981 tggctggacg ccaacacctt ggccgagaag gacgagtttg agcacaagag gaaggagctg
2041 gagcaggtgt gtaaccccat catcagcgga ctgtaccagg gtgccggtgg tcccgggcct
2101 gggggcttcg gggctcaggg tcccaaggga gggtctgggt caggccccac cattgaggag
2161 gtagattagg ggcctttcca agattgctgt ttttgttttg gagcttcaag actttgcatt
2221 tcctagtatt tctgtttgtc agttctcaat ttcctgtgtt tgcaatgttg aaattttttg
2281 gtgaagtact gaacttgctt tttttccggt ttctacatgc agagatgaat ttatactgcc
2341 atcttacgac tatttcttct ttttaataca cttaactcag gccatttttt aagttggtta
2401 cttcaaagta aataaacttt aaaattcaaa aaaaaaaaaa aaaaa
SEQ ID NO: 4: The protein sequence for Homo sapiens heat shock 70k Da protein 1B (HSPA1B_HUMAN) (NM_005346.4 / UniProtKB - P0DMV9): 
MAKAAAIGIDLGTTYSCVGVFQHGKVEIIANDQGNRTTPSYVAFTDTERLIGDAAKNQVALNPQNTVFDA
KRLIGRKFGDPVVQSDMKHWPFQVINDGDKPKVQVSYKGETKAFYPEEISSMVLTKMKEIAEAYLGYPVT
NAVITVPAYFNDSQRQATKDAGVIAGLNVLRIINEPTAAAIAYGLDRTGKGERNVLIFDLGGGTFDVSIL
TIDDGIFEVKATAGDTHLGGEDFDNRLVNHFVEEFKRKHKKDISQNKRAVRRLRTACERAKRTLSSSTQA
 
SLEIDSLFEGIDFYTSITRARFEELCSDLFRSTLEPVEKALRDAKLDKAQIHDLVLVGGSTRIPKVQKLL
QDFFNGRDLNKSINPDEAVAYGAAVQAAILMGDKSENVQDLLLLDVAPLSLGLETAGGVMTALIKRNSTI
PTKQTQIFTTYSDNQPGVLIQVYEGERAMTKDNNLLGRFELSGIPPAPRGVPQIEVTFDIDANGILNVTA
TDKSTGKANKITITNDKGRLSKEEIERMVQEAEKYKAEDEVQRERVSAKNALESYAFNMKSAVEDEGLKG
KISEADKKKVLDKCQEVISWLDANTLAEKDEFEHKRKELEQVCNPIISGLYQGAGGPGPGGFGAQGPKGG
SGSGPTIEEVD
SEQ ID NO: 5: The initiator methionine (M at position 1) of SEQ ID NO:4 is removed to yield a 640-amino acid long sequence (position 2-641): 
AKAAAIGIDLGTTYSCVGVFQHGKVEIIANDQGNRTTPSYVAFTDTERLIGDAAKNQVALNPQNTVFDAK
RLIGRKFGDPVVQSDMKHWPFQVINDGDKPKVQVSYKGETKAFYPEEISSMVLTKMKEIAEAYLGYPVTN
AVITVPAYFNDSQRQATKDAGVIAGLNVLRIINEPTAAAIAYGLDRTGKGERNVLIFDLGGGTFDVSILT
IDDGIFEVKATAGDTHLGGEDFDNRLVNHFVEEFKRKHKKDISQNKRAVRRLRTACERAKRTLSSSTQAS
LEIDSLFEGIDFYTSITRARFEELCSDLFRSTLEPVEKALRDAKLDKAQIHDLVLVGGSTRIPKVQKLLQ
DFFNGRDLNKSINPDEAVAYGAAVQAAILMGDKSENVQDLLLLDVAPLSLGLETAGGVMTALIKRNSTIP
TKQTQIFTTYSDNQPGVLIQVYEGERAMTKDNNLLGRFELSGIPPAPRGVPQIEVTFDIDANGILNVTAT
DKSTGKANKITITNDKGRLSKEEIERMVQEAEKYKAEDEVQRERVSAKNALESYAFNMKSAVEDEGLKGK
ISEADKKKVLDKCQEVISWLDANTLAEKDEFEHKRKELEQVCNPIISGLYQGAGGPGPGGFGAQGPKGGS
GSGPTIEEVD
SEQ ID NO: 6 The nucleic acid (DNA) sequence for Homo sapiens heat shock 70kDa protein 1B (HSPA1B) (NM_005346.4):

1 ggaaaacggc cagcctgagg agctgctgcg agggtccgct tcgtctttcg agagtgactc
61 ccgcggtccc aaggctttcc agagcgaacc tgtgcggctg caggcaccgg cgtgttgagt
121 ttccggcgtt ccgaaggact gagctcttgt cgcggatccc gtccgccgtt tccagccccc
181 agtctcagag cggagcccac agagcagggc accggcatgg ccaaagccgc ggcgatcggc
241 atcgacctgg gcaccaccta ctcctgcgtg ggggtgttcc aacacggcaa ggtggagatc
301 atcgccaacg accagggcaa ccgcaccacc cccagctacg tggccttcac ggacaccgag
361 cggctcatcg gggatgcggc caagaaccag gtggcgctga acccgcagaa caccgtgttt
421 gacgcgaagc ggctgatcgg ccgcaagttc ggcgacccgg tggtgcagtc ggacatgaag
481 cactggcctt tccaggtgat caacgacgga gacaagccca aggtgcaggt gagctacaag
541 ggggagacca aggcattcta ccccgaggag atctcgtcca tggtgctgac caagatgaag
601 gagatcgccg aggcgtacct gggctacccg gtgaccaacg cggtgatcac cgtgccggcc
661 tacttcaacg actcgcagcg ccaggccacc aaggatgcgg gtgtgatcgc ggggctcaac
721 gtgctgcgga tcatcaacga gcccacggcc gccgccatcg cctacggcct ggacagaacg
781 ggcaaggggg agcgcaacgt gctcatcttt gacctgggcg ggggcacctt cgacgtgtcc
841 atcctgacga tcgacgacgg catcttcgag gtgaaggcca cggccgggga cacccacctg
901 ggtggggagg actttgacaa caggctggtg aaccacttcg tggaggagtt caagagaaaa
961 cacaagaagg acatcagcca gaacaagcga gccgtgaggc ggctgcgcac cgcctgcgag

1021 agggccaaga ggaccctgtc gtccagcacc caggccagcc tggagatcga ctccctgttt
1081 gagggcatcg acttctacac gtccatcacc agggcgaggt tcgaggagct gtgctccgac
1141 ctgttccgaa gcaccctgga gcccgtggag aaggctctgc gcgacgccaa gctggacaag
1201 gcccagattc acgacctggt cctggtcggg ggctccaccc gcatccccaa ggtgcagaag
1261 ctgctgcagg acttcttcaa cgggcgcgac ctgaacaaga gcatcaaccc cgacgaggct
1321 gtggcctacg gggcggcggt gcaggcggcc atcctgatgg gggacaagtc cgagaacgtg
1381 caggacctgc tgctgctgga cgtggctccc ctgtcgctgg ggctggagac ggccggaggc
1441 gtgatgactg ccctgatcaa gcgcaactcc accatcccca ccaagcagac gcagatcttc
1501 accacctact ccgacaacca acccggggtg ctgatccagg tgtacgaggg cgagagggcc
1561 atgacgaaag acaacaatct gttggggcgc ttcgagctga gcggcatccc tccggccccc
1621 aggggcgtgc cccagatcga ggtgaccttc gacatcgatg ccaacggcat cctgaacgtc
1681 acggccacgg acaagagcac cggcaaggcc aacaagatca ccatcaccaa cgacaagggc
1741 cgcctgagca aggaggagat cgagcgcatg gtgcaggagg cggagaagta caaagcggag
1801 gacgaggtgc agcgcgagag ggtgtcagcc aagaacgccc tggagtccta cgccttcaac
1861 atgaagagcg ccgtggagga tgaggggctc aagggcaaga tcagcgaggc ggacaagaag
1921 aaggttctgg acaagtgtca agaggtcatc tcgtggctgg acgccaacac cttggccgag
1981 aaggacgagt ttgagcacaa gaggaaggag ctggagcagg tgtgtaaccc catcatcagc
2041 ggactgtacc agggtgccgg tggtcccggg cctggcggct tcggggctca gggtcccaag
2101 ggagggtctg ggtcaggccc taccattgag gaggtggatt aggggccttt gttctttagt
2161 atgtttgtct ttgaggtgga ctgttgggac tcaaggactt tgctgctgtt ttcctatgtc
2221 atttctgctt cagctctttg ctgcttcact tctttgtaaa gttgtaacct gatggtaatt
2281 agctggcttc attatttttg tagtacaacc gatatgttca ttagaattct ttgcatttaa
2341 tgttgatact gtaagggtgt ttcgttccct ttaaatgaat caacactgcc accttctgta
2401 cgagtttgtt tgtttttttt tttttttttt ttttttgctt ggcgaaaaca ctacaaaggc
2461 tgggaatgta tgtttttata atttgtttat ttaaatatga aaaataaaat gttaaacttt
2521 aaaaaaaaaa aaaaaaaaaa aaaaaaaaaa a

### Examples

### Example 1: Determining Hsp70 level in PBMC samples

The level of Hsp70 protein in PBMC samples isolated from healthy individuals was compared to Hsp70 levels in PBMC samples from individuals with Niemann Pick disease Type C.

### Introduction

Niemann Pick disease Type C (NPC) is a rare devastating neurodegenerative disease, caused by mutations in either the NPC1 (95% of cases) or NPC2 genes. While NPC1 is a lysosomal/endosomal membrane protein, NPC2 is a soluble cholesterol binding lysosomal protein, and together they play essential roles in lysosomal biogenesis. NPC disease is characterized by an enlarged, dysfunctional lysosomal compartment and aberrant accumulation of cholesterol and glycosphingolipids (GSL) inside the cells of multiple tissues causing the pathology of the disease (Platt, 2014).

Hsp70, an evolutionary conserved protein, stabilizes the lysosome by directed interaction with lysosomal proteins (T Kirkegaard et al., 2010). Therefore, Hsp70 therapies were considered as a potential treatment for NPC. Correspondingly, administration of Hsp70 or arimoclomol, a well-known inducer of the heat shock response, reduced the size of the lysosomal compartment and reversed the neuronal as well as visceral pathology of mutant NPC1 mice (Thomas Kirkegaard et al., 2016). Furthermore, the endogenous level of Hsp70 was found to be reduced in the brain and liver of the NPC1 mice.

A prospective non-interventional clinical study in NPC patients was initiated prior to initiation of a placebo controlled study with arimoclomol. Patients, 2 to 18 years of age, diagnosed with NPC, who had at least one neurological symptom and who had preserved ability to walk with assistance were eligible. Patients were maintained on standard therapy. All data were evaluated at inclusion (visit 1) and after 6 to 14 months (visit 2) of prospective observation. Disease severity was determined using the NPC-severity scale score (Yanjanin et al., 2010), where a higher score is associated with a more severe disease.

To gain a better understanding the disease pathology, we set out to determine Hsp70 in untreated patients. However, neither brain nor liver is an appropriate matrix for NPC patients. At the time of the planning of the clinical study a paper describing the classical NPC pathology in PBMC had just been published (Te Vruchte et al., 2014). We therefore found PBMC as a relevant matrix for determination of Hsp70 in NPC patients.

### Methods

### Sample collection:

Whole blood samples were collected in 6 mL EDTA tubes at the clinical study sites and shipped ambient to a central laboratory for sample preparation.

Clinical study samples (n=26) were obtained from individuals with Niemann Pick disease Type C (NPC patients), who were 2 to 18 years of age, diagnosed with NPC, had at least one neurological symptom and had preserved ability to walk with assistance. Patients were maintained on standard therapy during the study. Samples were collected at a first clinical study visit (inclusion) and at a second clinical study visit 6 to 14 months following the first visit.

Control samples (n=19) were obtained from healthy subjects at age 20 to 30. These samples were handled using the same collection and separation methods as the clinical study samples.

### NPC-severity scale score:

At the first and second clinical study visit, disease severity of NPC patients was determined using the NPC-severity scale score (NPCCSS) (Yanjanin et al., 2010), where a higher score is associated with a more severe disease.

### Separation of PBMCs:

Plasma was separated from whole blood by centrifugation at room temperature. Blood volume was restored in PBS and PBMCs were isolated by density gradient centrifugation using Histopaque^{™}. Viable PBMC cell count was determined by flow cytometry. Aliquots of 1 mio. PBMC were generated and stored frozen at -70 °C.

### Determination of Hsp70 in human PBMC:

Hsp70 was quantified using the Human/Mouse/Rat total HSP70 DuoSet^{®} IC ELISA kit (R&D Systems). Prior to analysis of the clinical study samples, the analytical method had been qualified for determination of Hsp70 in human PBMC samples. The Human/Mouse/Rat total HSP70 DuoSet^{®} IC ELISA kit detects the levels of HspA1A and HspA1B with no cross-reactivity of HspA5 and HspA8.

### Results

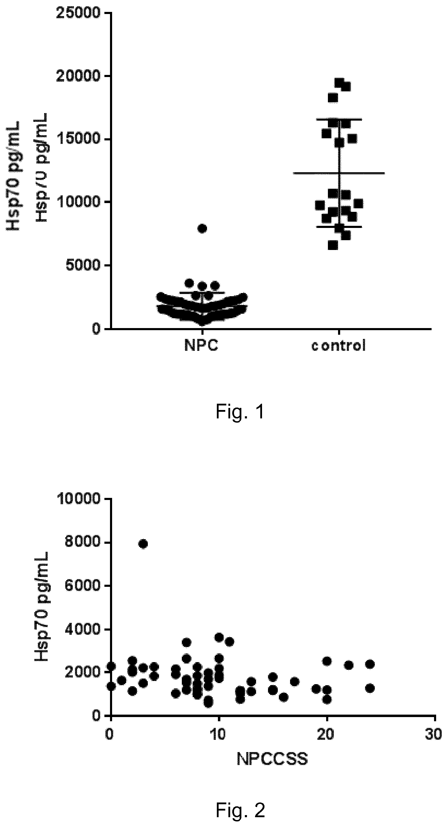

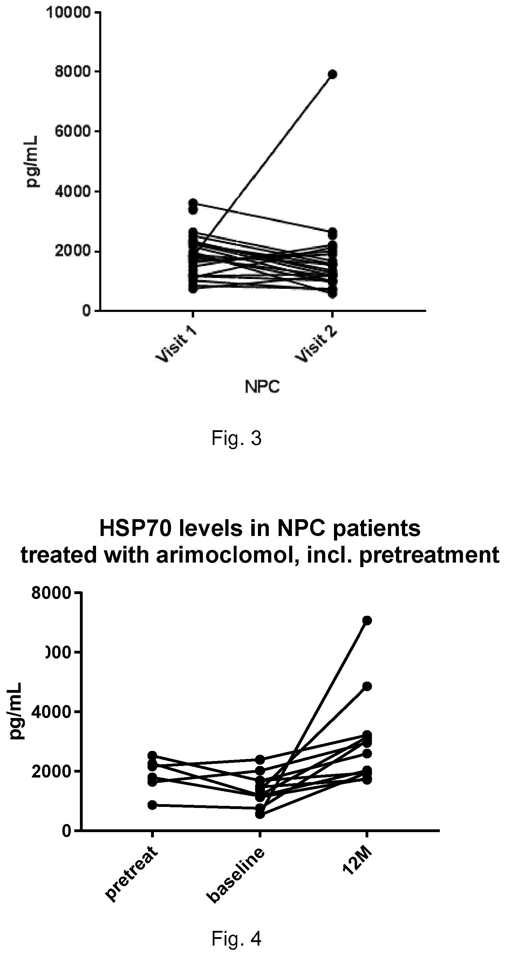

Quantification of Hsp70 in the PBMC samples showed a markedly reduced expression of Hsp70 in the PBMC samples derived from NPC patients compared to the PBMC samples from healthy controls. The healthy control samples showed an average of 12000 pg/mL of Hsp70, whereas the average concentration of Hsp70 in the PBMC samples isolated from NPC patients was 1800 pg/mL (Figure 1). The expression of Hsp70 in PBMC samples isolated from NPC patients showed no correlation with the NPC-severity scale score (NPCCSS) (figure 2). Furthermore, no change in Hsp70 level over the time from clinical study visit 1 to visit 2 was observed (Figure 3).

### Conclusion

The example demonstrates that Hsp70 protein expression is markedly reduced in Peripheral Blood mononuclear cells (PBMC) in patients suffering from Niemann Pick Type C as compared to healthy controls. Furthermore, the low level of Hsp70 is independent of the disease severity of the NPC patients.

### Example 2: Arimoclomol increases Hsp70 in NPC patients

### Materials & Methods

The materials and methods were performed as described in Example 1.

### Results

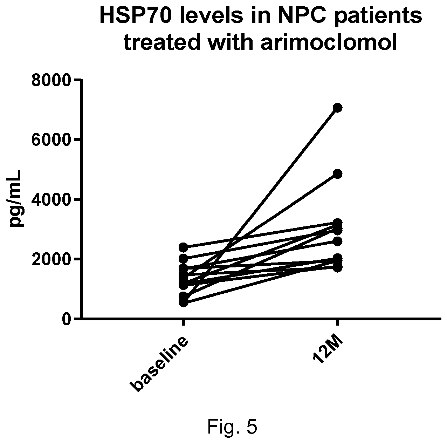

The Hsp70 concentration was measured in homogenates of Peripheral Blood Mononuclear Cells (PBMC). Hsp70 was determined in an observational study (Figures 4 and 5), at screening (pretreatment) and then again after 6-14 months (baseline). Patients were treated for 12 months with arimoclomol (Clinicaltrials.gov identifier NCT02612129). Hsp70 levels were analyzed by the Wilcoxon signed rank test for the 12 patients on arimoclomol with baseline and 12 months data point comparing the Hsp70 levels before and after 12 months of treatment.

**Table 2: Statistical analysis of retrieved data.**

| Change from baseline (pg/mL) arimoclomol | |
|---|---|
| n | 12 |
| mean (SD) | 1815.0 (1754.6) |
| Median | 1175.5 |
| Min - max | 234.7-6509.0 |
| Paired t-test p-value | 0.0043 |
| Wilcoxon signed rank test p-value | 0.0005 |

### Conclusion

Hsp70 blood levels are reduced in patients with NPC, compared to healthy controls, and remains stable with disease progression (from pretreat to baseline). This example demonstrates that treatment with arimoclomol for 12 months significantly increased the PBMC-level of Hsp70 compared to baseline. This demonstrates that the PBMC-level of Hsp70 is a pharmacodynamic marker for arimoclomol therapy.

### References

Kirkegaard, T., Gray, J., Priestman, D. A., Wallom, K., Atkins, J., Olsen, O. D., ... Platt, F. M. (2016). Heat shock protein-based therapy as a potential candidate for treating the sphingolipidoses. Science Translational Medicine, 8(355), 355ra118. https://doi.org/10.1126/scitranslmed.aad9823
Kirkegaard, T., Roth, A. G., Petersen, N. H. T., Mahalka, A. K., Olsen, O. D., Moilanen, I., ... Jäättelä, M. (2010). Hsp70 stabilizes lysosomes and reverts Niemann-Pick disease-associated lysosomal pathology. Nature, 463(7280), 549-53. https://doi.org/10.1038/nature08710
Platt, F. M. (2014). Sphingolipid lysosomal storage disorders. Nature, 510(7503), 68-75. https://doi.org/10.1038/nature13476
Te Vruchte, D., Speak, A. O., Wallom, K. L., Al Eisa, N., Smith, D. A., Hendriksz, C. J., ... Platt, F. M. (2014). Relative acidic compartment volume as a lysosomal storage disorder-associated biomarker. The Journal of Clinical Investigation, 1-9. https://doi.org/10.1172/LCI72835
Yanjanin, N. M., Vélez, J. I., Sc, M., Gropman, A., King, K., Au, D., ... Porter, F. D. (2010). Linear Clinical Progression, Independent of Age of Onset, in Niemann-Pick Disease, type C. American Journal Of Medical Genetics Part B Neuropsychiatric Genetics, (1), 132-140. https://doi.org/10.1002/ajmg.b.30969.Linear

## Claims

1. A method for diagnosing a disease selected from Niemann Pick Disease, Type C, (NPC) and Amyotrophic Lateral Sclerosis (ALS), in an individual, said method comprising the steps of:
a) providing a PBMC sample obtained from said individual,
b) detecting Hsp70 in said PBMC sample, and
c) quantifying or determining the level of Hsp70 in said PBMC sample,
wherein said Hsp70 is HspA1A and HspA1B.

2. The method according to claim 1 further comprising the step of
d) classifying or determining whether or not the individual has, or is likely to have, a disease selected from NPC and ALS.

3. The method according to claim 2, wherein said step d) comprises determining the level of HspA1A and HspA1B in the PBMC sample as compared to the level in a PBMC sample from a healthy control.

4. The method according to any one of the preceding claims, wherein the individual has, or is likely to have, a disease selected from NPC and ALS if the level of HspA1A and HspA1B in the PBMC sample is 1 to 1000 times lower than the level found in healthy controls,
such as 1 to 2 times, 2 to 3 times, 3 to 4 times, 4 to 5 times, 5 to 6 times, 6 to 7 times, 7 to 8 times, 8 to 9 times, 9 to 10 times, 10 to 11 times, 11 to 12 times, 12 to 13 times, 13 to 14 times, 14 to 15 times, 15 to 16 times, 16 to 17 times, 17 to 18 times, 18 to 19 times, 19 to 20 times, 20 to 25 times, 25 to 30 times, 30 to 35 times, 35 to 40 times, 40 to 45 times, 45 to 50 times, 50 to 75 times, 75 to 100 times, 100 to 150 times, 150 to 200 times, 200 to 250 times, 250 to 300 times, 300 to 400 times, 400 to 500 times, 500 to 750 times, 750 to 1000 times lower than the level found in a healthy control, or is undetectable.

5. The method according to any one of claims 3 and 4, wherein said step d) of classifying or determining an individual as having, or likely to have, a disease selected from NPC and ALS comprises determining if the amount of HspA1A and HspA1B in said PBMC sample is below a predefined cut-off value, or is undetectable.

6. The method according to any one of the preceding claims, wherein the individual has, or is likely to have, a disease selected from NPC and ALS if the amount of HspA1A and HspA1B in said PBMC sample is 7500 pg/mL or less, such as 7000 pg/mL or less, such as 6500 pg/mL or less, such as 6000 pg/mL or less, such as 5500 pg/mL or less, such as 5000 pg/mL or less, such as 4500 pg/mL or less, such as 4000 pg/mL or less, such as 3500 pg/mL or less, such as 3000 pg/mL or less, such as 2500 pg/mL or less, such as 2000 pg/mL or less, such as 1500 pg/mL or less, such as 1000 pg/mL or less.

7. The method according to any one of claims 5 and 6, wherein the individual is likely, or more likely, to respond to therapies for treatment of a disease selected from NPC and ALS if the amount of HspA1A and HspA1B is below said cut-off value.

8. A method for monitoring disease progression in an individual having a disease selected from NPC and ALS, said method comprising the steps of
a) providing one or more PBMC samples obtained from said individual at two or more subsequent points in time,
b) detecting Hsp70 in each of said PBMC samples, and
c) quantifying or determining the level of Hsp70 in each of said PBMC samples,
wherein said Hsp70 is HspA1A and HspA1B.

9. A method for monitoring efficacy of a therapy for treatment of a disease selected from NPC and ALS in an individual having a disease selected from NPC and ALS, said method comprising the steps of
a) providing one or more PBMC samples obtained from said individual before, during and/or after said therapy has been applied, maintained, reduced or elevated,
b) detecting Hsp70 in each of said one or more PBMC samples, and
c) quantifying or determining the level of Hsp70 in each of said one or more PBMC samples,
wherein said Hsp70 is HspA1A and HspA1B.

10. The method according to any one of the preceding claims, wherein said detecting HspA1A and HspA1B comprises detecting HspA1A and HspA1B protein, and/or wherein said method comprises detecting HspA1A and HspA1B RNA.

11. The method according to any one of claims 7 and 9, wherein a therapy for treatment of a disease selected from NPC and ALS is a bioactive agent that increase the intracellular concentration and/or activity of heat shock proteins, including Hsp70.

12. The method according to claim 11, wherein said bioactive agent that increases the intracellular concentration and/or activity of heat shock proteins, including Hsp70, is selected from the group consisting of arimoclomol, iroxanadine, bimoclomol, BGP-15, their stereoisomers and the acid addition salts thereof.

13. The method according to any one of claims 7 and 9, wherein a therapy for treatment of a disease selected from NPC and ALS is arimoclomol, its stereoisomers and the acid addition salts thereof.

14. The method according to any one of the preceding claims, wherein said disease is Niemann Pick Disease, Type C (NPC).

15. The method according to any one of claims 7 and 9, wherein a therapy for treatment of NPC is selected from arimoclomol, its stereoisomers and the acid addition salts thereof; Ambroxol and Miglustat.

## Patentansprüche

1. Verfahren zur Diagnose einer aus Niemann-Pick-Krankheit Typ C (NPC) und Amyotropher Lateralsklerose (ALS) ausgewählten Krankheit bei einem Individuum, umfassend die Schritte:
a) Bereitstellen einer von dem Individuum erhaltenen PBMC-Probe,
b) Nachweisen von Hsp70 in der PBMC-Probe, und
c) Quantifizieren oder Bestimmen des Gehalts an Hsp70 in der PBMC-Probe, wobei das Hsp70 HspA1A und HspA1B ist.

2. Verfahren nach Anspruch 1, zusätzlich umfassend den Schritt
d) Klassifizieren oder Bestimmen, ob das Individuum eine aus NPC und ALS ausgewählte Krankheit hat oder wahrscheinlich hat.

3. Verfahren nach Anspruch 2, wobei der Schritt d) das Bestimmen des Gehalts an HspA1A und HspA1B in der PBMC-Probe im Vergleich zu dem Gehalt in einer PBMC-Probe eines gesunden Kontrollindividuums umfasst.

4. Verfahren nach einem der vorhergehenden Ansprüche, wobei das Individuum eine aus NPC und ALS ausgewählte Krankheit hat oder wahrscheinlich hat, wenn der Gehalt an HspA1A und HspA1B in der PBMC-Probe 1- bis 1000-fach niedriger ist als der bei gesunden Kontrollindividuen gefundene Gehalt,
wie 1- bis 2-fach, 2- bis 3-fach, 3- bis 4-fach, 4- bis 5-fach, 5- bis 6-fach, 6- bis 7-fach, 7- bis 8-fach, 8- bis 9-fach, 9- bis 10-fach, 10- bis 11-fach, 11- bis 12-fach, 12-bis 13-fach, 13- bis 14-fach, 14- bis 15-fach, 15- bis 16-fach, 16- bis 17-fach, 17- bis 18-fach, 18- bis 19-fach, 19- bis 20-fach, 20- bis 25-fach, 25- bis 30-fach, 30- bis 35-fach, 35- bis 40-fach, 40- bis 45-fach, 45- bis 50-fach, 50- bis 75-fach, 75- bis 100-fach, 100- bis 150-fach, 150- bis 200-fach, 200- bis 250-fach, 250- bis 300-fach, 300- bis 400-fach, 400- bis 500-fach, 500- bis 750-fach, 750- bis 1000-fach niedriger ist als der bei einem gesunden Kontrollindividuum gefundene Gehalt, oder wenn der Gehalt nicht nachweisbar ist.

5. Verfahren nach einem der Ansprüche 3 und 4, wobei der Schritt d) des Klassifizierens oder Bestimmens ob ein Individuum ein aus NPC und ALS ausgewähltes Krankheitsbild hat oder wahrscheinlich hat umfasst, zu bestimmen, ob die Menge an HspA1A und HspA1B in der PBMC-Probe unterhalb eines vorgegebenen Grenzwerts liegt, oder nicht nachweisbar ist.

6. Verfahren nach einem der vorhergehenden Ansprüche, wobei das Individuum eine aus NPC und ALS ausgewählte Krankheit hat oder wahrscheinlich hat, wenn die Menge an HspA1A und HspA1B in der PBMC-Probe 7500 pg/mL oder weniger beträgt, wie 7000 pg/mL oder weniger, 6500 pg/mL oder weniger, 6000 pg/mL oder weniger, 5500 pg/mL oder weniger, 5000 pg/mL oder weniger, 4500 pg/mL oder weniger, 4000 pg/mL oder weniger, 3500 pg/mL oder weniger, 3000 pg/mL oder weniger, 2500 pg/mL oder weniger, 2000 pg/mL oder weniger, 1500 pg/mL oder weniger oder 1000 pg/mL oder weniger.

7. Verfahren nach einem der Ansprüche 5 und 6, wobei das Individuum wahrscheinlich oder wahrscheinlicher auf Therapien zur Behandlung einer aus NPC und ALS ausgewählten Krankheit anspricht, wenn die Menge an HspA1A und HspA1B unter dem Grenzwert liegt.

8. Verfahren zur Überwachung des Krankheitsverlaufs bei einem Individuum mit einer aus NPC und ALS ausgewählten Krankheit, umfassend die Schritte:
a) Bereitstellen einer oder mehrerer PBMC-Proben, die von dem Individuum zu zwei oder mehr aufeinanderfolgenden Zeitpunkten erhalten wurden,
b) Nachweisen von Hsp70 in jeder der PBMC-Proben und
c) Quantifizieren oder Bestimmen des Gehalts an Hsp70 in jeder der PBMC-Proben,
wobei das Hsp70 HspA1A und HspA1B ist.

9. Verfahren zur Überwachung der Wirksamkeit einer Therapie zur Behandlung einer aus NPC und ALS ausgewählten Krankheit bei einem Individuum mit einer solchen Krankheit, umfassend die Schritte:
a) Bereitstellen einer oder mehrerer PBMC-Proben, die von dem Individuum vor, während und/oder nach dem Anwenden, Aufrechterhalten, Reduzieren oder Erhöhen der Therapie erhalten wurden,
b) Nachweisen von Hsp70 in jeder der PBMC-Proben und
c) Quantifizieren oder Bestimmen des Gehalts an Hsp70 in jeder der PBMC-Proben,
wobei das Hsp70 HspA1A und HspA1B ist.

10. Verfahren nach einem der vorhergehenden Ansprüche, wobei das Nachweisen von HspA1A und HspA1B das Nachweisen von HspA1A- und HspA1B-Protein umfasst und/oder wobei das Verfahren das Nachweisen von HspA1A- und HspA1B-RNA umfasst.

11. Verfahren nach einem der Ansprüche 7 und 9, wobei eine Therapie zur Behandlung einer aus NPC und ALS ausgewählten Krankheit ein bioaktiver Wirkstoff ist, der die intrazelluläre Konzentration und/oder Aktivität von Hitzeschockproteinen, einschließlich Hsp70, erhöht.

12. Verfahren nach Anspruch 11, wobei der bioaktive Wirkstoff, der die intrazelluläre Konzentration und/oder Aktivität von Hitzeschockproteinen einschließlich Hsp70 erhöht, aus der Gruppe ausgewählt ist, umfassend Arimoclomol, Iroxanadin, Bimoclomol, BGP-15, deren Stereoisomere und deren Säureadditionssalze.

13. Verfahren nach einem der Ansprüche 7 und 9, wobei eine Therapie zur Behandlung einer aus NPC und ALS ausgewählten Krankheit Arimoclomol, dessen Stereoisomere und deren Säureadditionssalze ist.

14. Verfahren nach einem der vorhergehenden Ansprüche, wobei die Krankheit Niemann-Pick-Krankheit Typ C (NPC) ist.

15. Verfahren nach einem der Ansprüche 7 und 9, wobei eine Therapie zur Behandlung von NPC ausgewählt ist aus Arimoclomol, dessen Stereoisomeren und deren Säureadditionssalzen; Ambroxol und Miglustat.

## Revendications

1. Procédé de diagnostic d'une maladie choisie parmi la maladie de Niemann-Pick de type C (NPC) et la sclérose latérale amyotrophique (SLA), chez un individu, ledit procédé comprenant les étapes consistant à :
a) fournir un échantillon de PBMC obtenu auprès dudit individu,
b) détecter Hsp70 dans ledit échantillon de PBMC, et
c) quantifier ou déterminer le niveau de Hsp70 dans ledit échantillon de PBMC,
dans lequel ledit Hsp70 est HspA1A et HspA1B.

2. Procédé selon la revendication 1 comprenant en outre l'étape consistant à
d) classifier ou déterminer si l'individu présente, ou est susceptible de présenter, une maladie choisie parmi NPC et SLA.

3. Procédé selon la revendication 2, dans lequel ladite étape d) comprend la détermination du niveau de HspA1A et HspA1B dans l'échantillon de PBMC par comparaison avec le niveau dans un échantillon de PBMC issu d'un témoin sain.

4. Procédé selon l'une quelconque des revendications précédentes, dans lequel l'individu présente, ou est susceptible de présenter, une maladie choisie parmi NPC et SLA si le niveau de HspA1A et HspA1B dans l'échantillon de PBMC est entre 1 et 1000 fois plus bas que le niveau trouvé chez des témoins sains,
tel que 1 à 2 fois, 2 à 3 fois, 3 à 4 fois, 4 à 5 fois, 5 à 6 fois, 6 à 7 fois, 7 à 8 fois, 8 à 9 fois, 9 à 10 fois, 10 à 11 fois, 11 à 12 fois, 12 à 13 fois, 13 à 14 fois, 14 à 15 fois, 15 à 16 fois, 16 à 17 fois, 17 à 18 fois, 18 à 19 fois, 19 à 20 fois, 20 à 25 fois, 25 à 30 fois, 30 à 35 fois, 35 à 40 fois, 40 à 45 fois, 45 à 50 fois, 50 à 75 fois, 75 à 100 fois, 100 à 150 fois, 150 à 200 fois, 200 à 250 fois, 250 à 300 fois, 300 à 400 fois, 400 à 500 fois, 500 à 750 fois, 750 à 1000 fois inférieur au niveau trouvé chez un témoin sain, ou est indétectable.

5. Procédé selon l'une quelconque des revendications 3 et 4, dans lequel ladite étape d) de classification ou de détermination qu'un individu présente, ou est susceptible de présenter, une maladie choisie parmi NPC et SLA comprend l'étape de determiner si la quantité de HspA1A et HspA1B dans ledit échantillon de PBMC est inférieure à une valeur seuil prédéfinie, ou est indétectable.

6. Procédé selon l'une quelconque des revendications précédentes, dans lequel l'individu présente, ou est susceptible de présenter, une maladie choisie parmi NPC et SLA si la quantité de HspA1A et HspA1B dans ledit échantillon de PBMC est de 7500 pg/mL ou moins, telle que 7000 pg/mL ou moins, telle que 6500 pg/mL ou moins, telle que 6000 pg/mL ou moins, telle que 5500 pg/mL ou moins, telle que 5000 pg/mL ou moins, telle que 4500 pg/mL ou moins, telle que 4000 pg/mL ou moins, telle que 3500 pg/mL ou moins, telle que 3000 pg/mL ou moins, telle que 2500 pg/mL ou moins, telle que 2000 pg/mL ou moins, telle que 1500 pg/mL ou moins, telle que 1000 pg/mL ou moins.

7. Procédé selon l'une quelconque des revendications 5 et 6, dans lequel l'individu est susceptible, ou plus susceptible, de répondre à des thérapies pour le traitement d'une maladie choisie parmi NPC et SLA si la quantité de HspA1A et HspA1B est inférieure à ladite valeur seuil.

8. Procédé de surveillance de la progression d'une maladie chez un individu ayant une maladie choisie parmi NPC et SLA, ledit procédé comprenant les étapes consistant à :
a) fournir un ou plusieurs échantillons de PBMC obtenus auprès dudit individu à deux ou plusieurs points dans le temps,
b) détecter Hsp70 dans chacun desdits échantillons de PBMC, et
c) quantifier ou déterminer le niveau de Hsp70 dans chacun desdits échantillons de PBMC,
dans lequel ledit Hsp70 est HspA1A et HspA1B.

9. Procédé de surveillance de l'efficacité d'une thérapie pour le traitement d'une maladie choisie parmi NPC et SLA chez un individu ayant une maladie choisie parmi NPC et SLA, ledit procédé comprenant les étapes consistant à :
a) fournir un ou plusieurs échantillons de PBMC obtenus auprès dudit individu avant, pendant et/ou après que ladite thérapie a été appliquée, maintenue, réduite ou augmentée,
b) détecter Hsp70 dans chacun desdits échantillons de PBMC, et
c) quantifier ou déterminer le niveau de Hsp70 dans chacun desdits échantillons de PBMC,
dans lequel ledit Hsp70 est HspA1A et HspA1B.

10. Procédé selon l'une quelconque des revendications précédentes, dans lequel ladite détection de HspA1A et HspA1B comprend la détection de la protéine HspA1A et HspA1B et/ou dans lequel ledit procédé comprend la détection de l'ARN de HspA1A et HspA1B.

11. Procédé selon l'une quelconque des revendications 7 et 9, dans lequel une thérapie pour le traitement d'une maladie choisie parmi NPC et SLA est un agent bioactif qui augmente la concentration intracellulaire et/ou l'activité des protéines de choc thermique, y compris Hsp70.

12. Procédé selon la revendication 11, dans lequel ledit agent bioactif qui augmente la concentration intracellulaire et/ou l'activité des protéines de choc thermique, y compris Hsp70, est choisi parmi arimoclomol, iroxanadine, bimoclomol, BGP-15, leurs stéréoisomères et leurs sels d'addition d'acide.

13. Procédé selon l'une quelconque des revendications 7 et 9, dans lequel une thérapie pour le traitement d'une maladie choisie parmi NPC et SLA est l'arimoclomol, ses stéréoisomères et ses sels d'addition d'acide.

14. Procédé selon l'une quelconque des revendications précédentes, dans lequel ladite maladie est la maladie de Niemann-Pick de type C (NPC).

15. Procédé selon l'une quelconque des revendications 7 et 9, dans lequel une thérapie pour le traitement de NPC est choisie parmi l'arimoclomol, ses stéréoisomères et ses sels d'addition d'acide ; l'Ambroxol et le Miglustat.
